# EUROPEAN PATENT SPECIFICATION

(11) **EP 2 733 205 B1**
(45) Date of publication and mention of the grant of the patent: **10.01.2018**
(21) Application number: 12382455.9
(22) Date of filing: 20.11.2012
(51) Int. Cl.: A61K 31/4439, A61K 35/30, C12N 5/0793, A61K 31/352, A61K 31/343

(54) **Corticospinal upper motor neurons, methods and compositions for differentiating neural stem cells by modulating CB1 cannabinoid receptor signaling and uses thereof**
Obere Pyramidenmotoneuronen, Verfahren und Zusammensetzungen zur Differenzierung neuraler Stammzellen durch Modulation der CB1-Cannabinoidrezeptor-Signalisierung und Verwendungen davon
Neurones moteurs supérieurs cortico-spinaux, procédés et compositions permettant de différencier des cellules souches neurales par modulation de la signalisation des récepteurs cannabinoïdes CB1 et leurs utilisations

(43) Date of publication of application: 21.05.2014
(60) Divisional application: 17192973.0
(73) Proprietor: Centro De Investigacíon Biomédica En Red De Enfermedades Neurodegenerativas Ciberned, 28031 Madrid (ES); Universidad Complutense De Madrid, 28040 Madrid (ES)
(72) Inventor: Galve-Roperh, Ismael, E-28024 Madrid (ES); Guzmán Pastor, Manuel, E-28015 Madrid (ES); Díaz-Alonso, Javier, E-35011 Las Palmas de Gran Canaria (ES); Aguado Sanchez, Tania, E-28660 Boadilla del Monte (ES); Paraíso Luna, Juan, 16411 Fuente de Pedro Naharro (Cuenca) (ES)
(74) Representative: ABG Patentes, S.L.

(56) References cited:
- EP-A1- 2 397 137
- SCHMIDT W ET AL: "CANNABINOID RECEPTOR SUBTYPES 1 AND 2 MEDIATE LONG-LASTING NEUROPROTECTION AND IMPROVE MOTOR BEHAVIOR DEFICITS AFTER TRANSIENT FOCAL CEREBRAL ISCHEMIA", NEUROSCIENCE, NEW YORK, NY, US, vol. 227, 13 October 2012 (2012-10-13), pages 313-326, XP009166815, ISSN: 0306-4522, DOI: 10.1016/J.NEUROSCIENCE.2012.09.080
- AGUADO TANIA ET AL: "The CB1 cannabinoid receptor mediates excitotoxicity-induced neural progenitor proliferation and neurogenesis", JOURNAL OF BIOLOGICAL CHEMISTRY, vol. 282, no. 33, August 2007 (2007-08), pages 23892-23898, XP9166809, ISSN: 0021-9258
- LICKISS TOM ET AL: "Examining the relationship between early axon growth and transcription factor expression in the developing cerebral cortex.", JOURNAL OF ANATOMY MAR 2012, vol. 220, no. 3, March 2012 (2012-03), pages 201-211, XP9166827, ISSN: 1469-7580
- PALAZUELOS JAVIER ET AL: "CB2 cannabinoid receptors promote neural progenitor cell proliferation via mTORC1 signaling", JOURNAL OF BIOLOGICAL CHEMISTRY, AMERICAN SOCIETY FOR BIOCHEMISTRY AND MOLECULAR BIOLOGY, INC, BETHESDA, MD, USA, vol. 287, no. 2, 6 January 2012 (2012-01-06), pages 1198-1209, XP009166803, ISSN: 1083-351X, DOI: 10.1074/JBC.M111.291294

## Description

### FIELD OF THE INVENTION

The present invention relates to methods and compositions for obtaining a corticospinal upper motor neuron from neural stem cells. Also described are the corticospinal upper motor neurons obtained and uses thereof.

### BACKGROUND OF THE INVENTION

Amyotrophic lateral sclerosis (ALS) is a devastating disorder characterized by degeneration of motor neurons. In particular, lower motor neuron degeneration has been extensively demonstrated in ALS and related disorders, but in addition upper motor neurons can also be affected (Ozdinler et al., 2011; Sach et al., 2004). Upper motor neurons are also involved in other motor neurodegenerative disorders (e.g. primary lateral sclerosis). Despite intense research, the origin and mechanisms responsible for selective motor neurodegeneration remain to be fully elucidated. Mutations in different genes including superoxide dismutase 1 (SOD1), TAR DNA binding protein-43 (TDP-43) and fus have been described to mimic some of the aspects of the pathology (Bruijn et al., 2004).

Current ALS approved treatments (e.g. riluzol) are only partially effective and thus there is an urgent need for alternative therapeutic approaches. Advances derived from the stem cell research field have succeeded to define protocols for the generation of functional cortical neurons from pluripotent stem cells (PSCs), particularly from embryonic stem cells (ES) and induced pluripotent stem cells (iPS) (Gaspard et al., 2008; Shi et al., 2012). Whereas PSC differentiation into the neuronal lineage results in the generation of cortical neurons with characteristics of both upper and lower layer neurons, the induction of a particular neuronal population is still limited to a minor fraction of cells. Thus, only a percentage of PSC-derived pyramidal neurons express markers characteristic of lower cortical layers in which corticospinal motor neurons are included.

Research in the ALS field has devoted most of its efforts to the efficient generation of lower motor neurons (LMNs) from PSCs and therefore has focused in ventral neuralization strategies (Wichterle et al., 2002; Chambers et al., 2009). This can be achieved by different protocols, among them: (i) neurogenic niche signal manipulation of iPS or ES comprising the differentiation of the embryoid bodies generated from said cells into spinal motor neurons by treating them with retinoic acid (RA) and a sonic hedgehog (Shh) agonist, such as purmorphamine (Dimos et al., 2008; Karumbayaram et al., 2009); and (ii) genetic manipulation of the motor neuron transcription factor program involving the use of an adenoviral gene delivery system encoding motor neuron inducing transcription factors, e.g. neurogenin 2 (Ngn2), islet-1 (Isl-1) and LIM/homeobox protein 3 (Lhx3) (Hester et al., 2011).
In contrast with the availability of well defined protocols for LMNs generation from PSCs (Chaddah et al., 2011), there are not at this stage good protocols for upper motor neuron (UMN) generation.

Therefore, there exists a long-felt and continuing need in the art for methods for obtaining corticospinal upper motor neurons from neural stem cells.

### SUMMARY OF THE INVENTION

In a first aspect, the invention relates to an *in vitro* method for obtaining a corticospinal upper motor neuron from a neural stem cell, wherein said method comprises contacting a neural stem cell under conditions adequate for increasing the signal mediated by the CB₁ cannabinoid receptor in said cell and under conditions to promote dorsalization of said cell. Also described is a corticospinal upper motor neuron obtainable by the method of the invention and to a cell population comprising corticospinal upper motor neurons of the invention. Also described is a pharmaceutical composition comprising a corticospinal upper motor neuron of the invention or a cell population of the invention, and a pharmaceutically acceptable carrier. Also described is the use of a corticospinal upper motor neuron of the invention or a cell population of the invention for evaluating a biological, pharmacological and/or chemical product. Also described is a corticospinal upper motor neuron of the invention, a cell population of the invention or a pharmaceutical composition of the invention for use as a medicament. Also described is a corticospinal upper motor neuron of the invention, a cell population of the invention or a pharmaceutical composition of the invention for use in the treatment of a motor neurodegenerative disorder.

In a further aspect, the invention relates to a composition comprising an agent capable of increasing the signal mediated by the CB₁ cannabinoid receptor in a neural stem cell and a further agent selected from: a) a sonic hedgehog inhibitor and b) a Smad inhibitor. Also described is an isolated neural stem cell having a loss of the CB₁ cannabinoid receptor function or to a population of neurons obtainable by *in vitro* differentiation from a neural stem cell having a loss of the CB₁ cannabinoid receptor function. Also described is the use of an isolated neural stem cell of the invention or a neuron of the invention as a model for studying a motor neurodegenerative disorder or for screening for compounds useful in the treatment of a motor neurodegenerative disorder.

### DESCRIPTION OF THE FIGURES

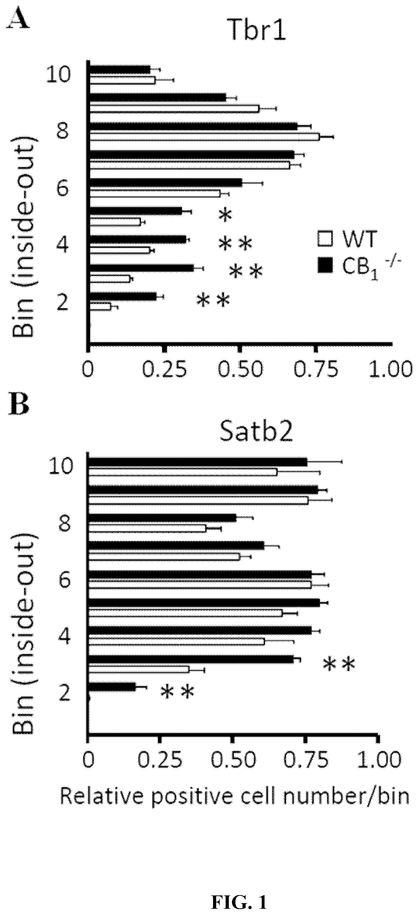

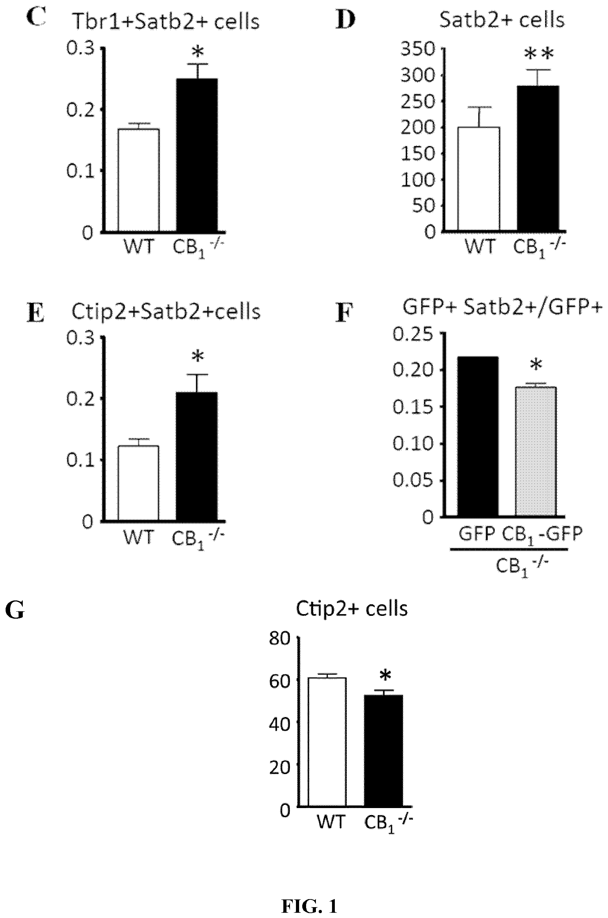

**Figure 1****.** CB₁ receptor inactivation interferes with the specification of upper- and deep-layer cortical neurons. Inmunofluorescence analysis was performed in CB₁^{-/-} mice and wild-type (WT) littermates at embryonic day E16.5 for Tbr1⁺, Satb2⁺, Ctip2⁺, Tbr1⁺Satb2⁺ and Ctip2⁺Satb2⁺ cells. **A, B:** Fraction of differentiating cells that express Tbr1 **(A)** and Satb2 **(B)** in CB₁^{-/-} and WT mice (black and white bars, respectively) quantified in equal-size binned areas and referred to total cell number (Hoechst 33528 counterstaining). **C, E:** Quantification of the neuronal cell fraction that coexpress Tbr1 with Satb2 **(C)** and Satb2 with Ctip2 **(E),** referred to total cell number (Hoechst 33528 counterstaining) in the cortical column of WT and CB₁^{-/-} mice (white and black columns, respectively). **D, G:** Satb2⁺ and Ctip2⁺ cells quantified in 50 µm-wide cortical columns of the same mice. **F:** *In utero* electroporation of E14.5-CB₁^{-/-} mice was performed with pCAG-GFP vector (GFP) and pCAG-CB₁-GFP vector (CB₁-GFP) to re-express CB₁ receptor (black and grey bars, respectively), and cortices were subsequently analyzed at embryonic day E16.5. Satb2⁺ cells in the GFP⁺ electroporated cell population were quantified. n= 5 for each group **(A-E),** n= 2 for each group **(F-G).** *, p <0.05; **, p <0.01 versus control mice.

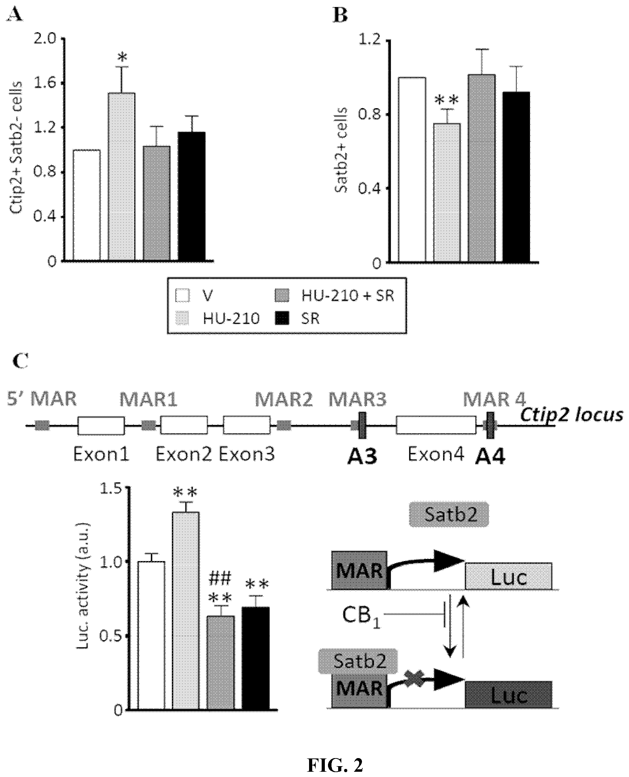

**Figure 2****.** CB₁ receptor signaling promotes Ctip2⁺ neuron differentiation. **A, B:** Immunofluorescence analysis was performed in cortical organotypic cultures from embryonic day E13.5 wild-type (WT) embryos treated for 3 days with vehicle (V), 5 µM HU-210, 25 µM SR141716 (SR), and a combination of 5 µM HU-210 and 25 µM SR141716 (HU-210 + SR). Fraction of Ctip2-only positive cells **(A)** and Satb2⁺ cells **(B)** quantified in the same conditions and referred to total cell number (Hoechst 33528 counterstaining). **C:** HiB5 neural stem cells were transfected with a luciferase reporter construct that contained the A4 MAR sequence of the Ctip2 locus together with pCAG-CB1 and pMSCV-Satb2. Ctip2-luciferase activity (Luc. Activity) was determined 36 h after treatment with vehicle (V), 50 nM HU-210, 1 µM SR141716 (SR), and a combination of 50 nM HU-210 and 1 µM SR141716 (HU-210 + SR). *, p <0.05; **, p <0.01 versus vehicle-treated cells; ## p <0.01 versus HU-210-treated cells. Results correspond to 4 independent experiments. a.u., arbitrary units; MAR, matrix attachment region; Luc, luciferase.

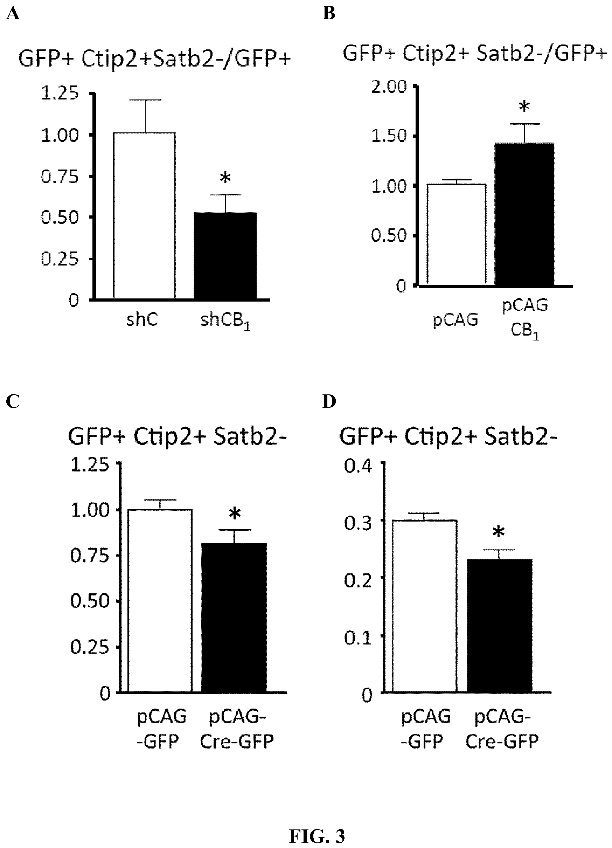

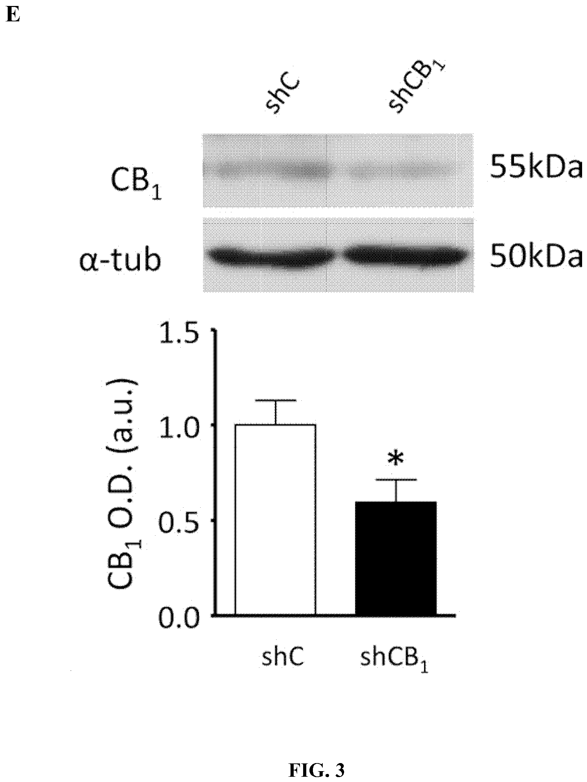

**Figure 3****.** Manipulation of CB₁ receptor expression controls Ctip2⁺ neuron differentiation. **A, B, E:** Primary cortical cultures obtained from embryonic day E13.5 wild-type (WT) cortices after *ex-utero* electroporation with shControl (shC), shCB₁, pCAG and PCAG-CB₁ were allowed to differentiate for 7 days *in vitro.* CB₁ receptor expression in electroporated cells was identified with GFP antibody. Ctip2-only positive cells were quantified in the electroporated GFP⁺ cell subpopulation after *ex-utero* electroporation with shControl (shC) and shCB₁ **(A),** and with pCAG and pCAG-CB₁ **(B). C:** CB₁ receptor expression was analyzed in electroporated cells (GFP⁺) in CB₁^{f/f}-derived cells after pCAG-GFP or pCAG-Cre-GFP electroporation of CB₁^{f/f} cortices. Ctip2-only-positive cells were quantified in the electroporated GFP+ cell subpopulation. **D:** *In utero* electroporation of E12.5 CB₁^{f/f} mice was performed with pCAG-GFP and pCAG-Cre-GFP vectors, and pups were subsequently analyzed at P0. Ctip2-only-positive cells that were electroporated (Ctip2⁺ Satb2⁻ GFP⁺) were quantified and the relative number to the GFP⁺ cell population is shown. n = 3 for each group. **E:** Western blot analysis of CB₁ receptor knowckdown after transfection of shControl (shC) and shCB₁ in P19 cells. α-tub, α-tubulin; O.D., optical density; a.u., arbitrary units. Results correspond to 4 independent experiments. *, p <0.05 versus control electroporated cells or pups. GFP, green fluorescent protein.

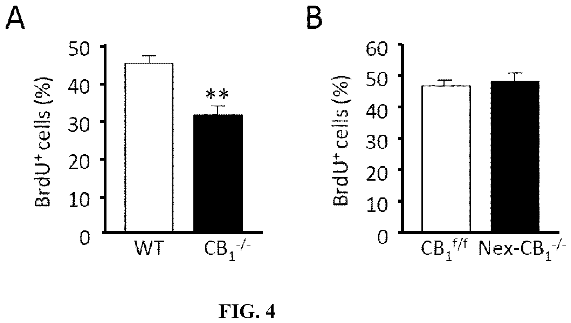

**Figure 4****.** Cortical progenitor cell proliferation is not affected in conditional Nex-CB₁^{-/-}mice. Quantification of progenitor cell proliferation as BrdU-labeled cells in the ventricular/subventricular zone (VZ/SVZ) of CB₁^{-/-} mice and wild-type (WT) littermates **(A),** and Nex-CB₁^{-/-} mice and CB₁^{f/f} littermates **(B),** at embryonic day E14.5. BrdU, 5-bromo-2'-deoxyuridine. **, p <0.01 versus control mice.

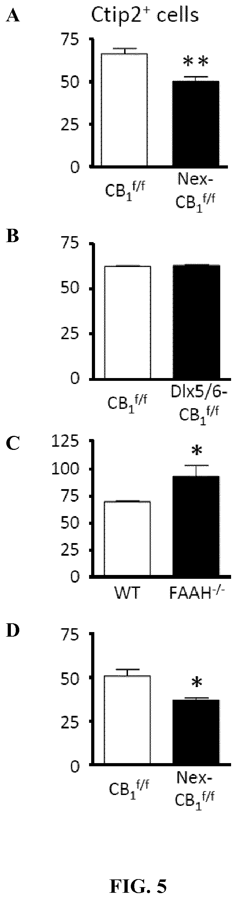

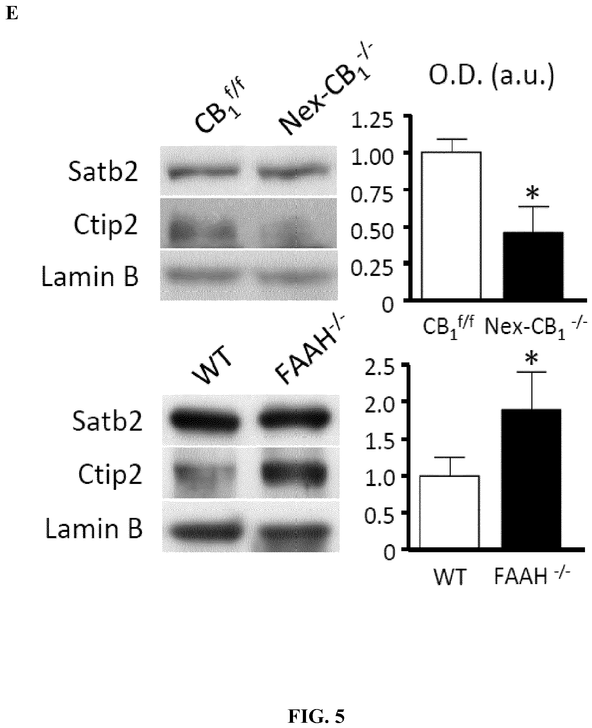

**Figure 5****.** CBi receptor signaling regulates the generation of deep-layer Ctip2⁺ neurons. **A-D:** Nex-CB₁^{-/-}, Dlx5/6-CB₁^{-/-} and FAAH^{-/-} mice and their respective wild-type (CB₁^{f/f} and WT) littermates were analyzed at P2 **(A-C)** and Ctip2⁺ neurons quantified in 50 µm-wide cortical columns (n= 8 and 9; 2 and 3; and 2 and 3, respectively for each group). Nex-CB₁^{-/-} and WT littermates were also analyzed at P8 **(D;** n= 4 for each group). **E:** Western blot analyses of nuclear extracts obtained from P2 cortices of Nex-CB₁^{-/-} and FAAH^{-/-} mice compared with their corresponding wild-type littermates. The relative protein levels of Ctip2 and Satb2 were quantified after densitometry and loading control was performed with anti-lamin B1 antibody.*, p<0.05; **, p <0.01 versus WT mice sections. WT, wild-type; P2, postnatal day 2; P8, postnatal day 8; O.D., optical density; a.u., arbitrary units.

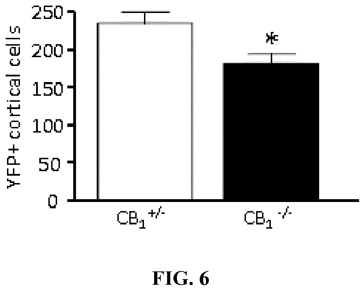

**Figure 6****.** CBi-deficient Thy1-YFP mice show alterations of subcerebral projection neurons. Fluorescent somata of putative CSMNs in layer 5 of sagittal sections of P21 cortices were obtained by confocal fluorescence from Thy1-YFP:CB₁^{+/-} (CB₁^{+/-}) and Thy1-YFP:CB₁^{-/-} (CB₁^{-/-}) littermates and were quantified. n=2 and 3, respectively. *, p <0.05. CSMNs, corticospinal motor neurons; P21, postnatal day 21; YFP: yellow fluorescent protein.

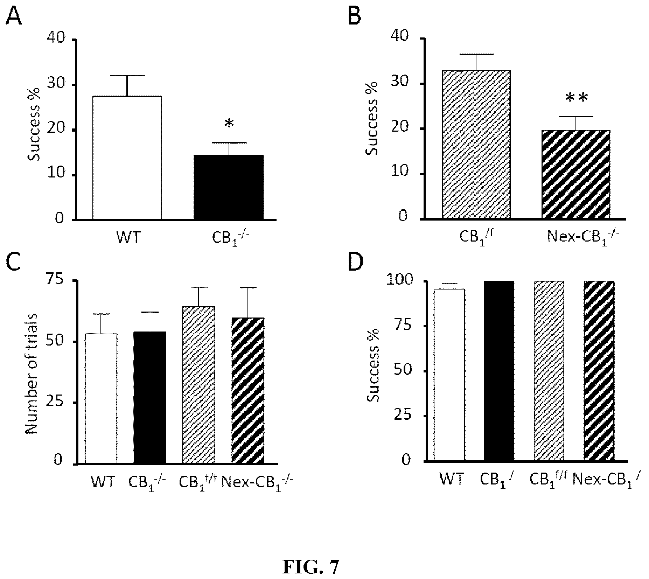

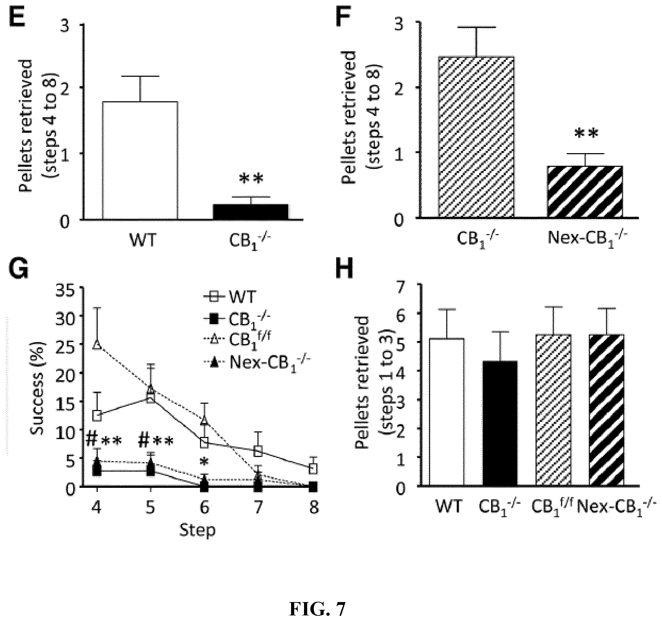

**Figure 7****.** CB₁-deficient mice show impaired corticospinal motor function. **A-C:** CB₁^{-/-}, Nex-CB₁^{-/-} and their corresponding wild-type littermates (WT and CB₁^{f/f}) were analyzed in the skilled pellet-reaching task test. Fine motor skill was evaluated in the animal groups after cage habituation and training. The percentage of pellets retrieved **(A, B)** and the total number of trials performed during the skilled task **(C)** are shown. **D:** Unskilled motor function was assessed and the percentage of pellets retrieved calculated. **E-F:** Mice were subjected to the staircase pellet-reaching test and the sum of pellets retrieved from challenging steps (from 4-8) was compared between CB₁^{-/-} or Nex-CB₁^{-/-} mice and their control littermates. **G:** The percentage of success for each step among the different genotypes was quantified. **H:** The number of pellets reached in non-challenging steps 1 to 3 did not differ between groups. n=9 (CB₁^{-/-} and WT) and n=17 and 16 (Nex-CB₁^{-/-} and CB₁^{f/f}) for each group. *, p <0.05 **; p <0.01 versus WT or CB₁^{f/f} littermates; # p <0.05 versus CB₁^{f/f} littermates.

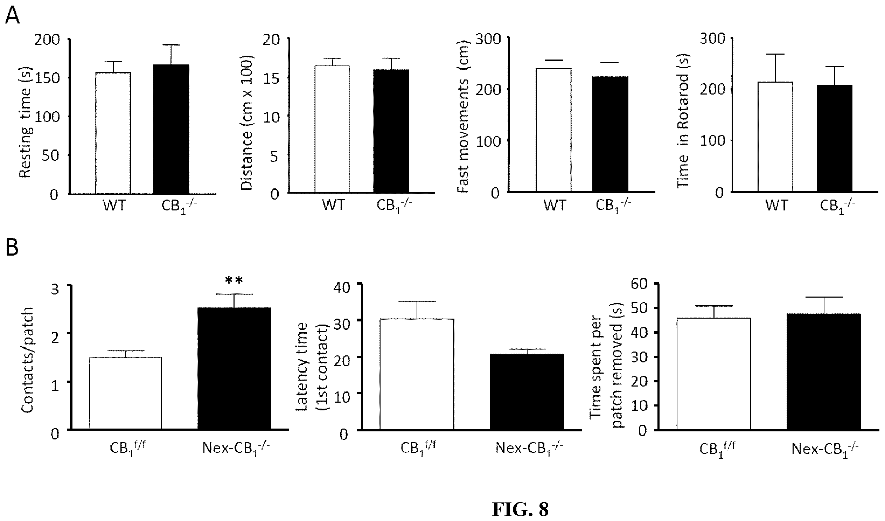

**Figure 8****.** Behavioral characterization of CB₁^{-/-} and Nex-CB₁^{-/-} mice. **A:** General motor behavior in CB₁^{-/-} mice and WT littermates. Adult mice were analyzed for ambulation in the open field test and for motor coordination in the RotaRod test. n= 9 for each group. **B:** Patch removal analysis confirms the deficient fine motor function of Nex-CB₁^{-/-} mice compared with their CB₁^{f/f} littermates. The number of contacts required for patch removal, the latency time for the first patch contact, and the time spent per patch were quantified in each genotype. n= 16 and 17 for each group. **, p <0.01 versus CB₁^{f/f} mice. WT, wild-type; s, seconds.

### DETAILED DESCRIPTION OF THE INVENTION

The authors of the present invention have surprisingly discovered that a CB₁ cannabinoid receptor agonist is capable of inducing the differentiation of cortical pyramidal neural progenitors to Ctip2-only positive cells (a marker of corticospinal upper motor neurons), while decreasing the number of Satb2⁺ cells (a repressor for the generation of upper motor neurons) organotypic cultures. These results indicate that a CB₁ cannabinoid receptor agonist is useful for obtaining a corticospinal upper motor neuron-enriched culture from neural stem cells. The authors of the present invention have also found that mice deficient in an endocannabinoid degrading enzyme (FAAH^{-/-}mice) showed an increased number in Ctip2⁺ cells through the cortex. Therefore, an inhibitor of an endocannabinoid-degrading enzyme is also useful for obtaining a corticospinal upper motor neuron-enriched culture from neural stem cells. These methods rely in the modulation of an endogenous receptor to improve corticospinal neuron generation and have the advantage that do not require gene manipulation. Methods involving gene manipulation may also be used, such as the modification of a neural stem cell to increase the expression of the CB₁ cannabinoid receptor above basal levels or to decrease the expression of an endocannabinoid-degrading enzyme under basal levels. The modification of a neural stem cell to increase the activity of an endocannabinoid-synthesizing enzyme above basal levels is also disclosed.

Corticospinal upper motor neurons obtained by the methods of the invention are useful for evaluating a biological, pharmacological and/or chemical product and also for the treatment of a motor neurodegenerative disorder.

The authors of the present invention have also shown that complete CB₁^{-/-} mice and glutamatergic neuron-specific Nex- CB₁^{-/-} mice have alterations in corticospinal motor neuron generation. Therefore, neural stem cells having a loss of the CB₁ cannabinoid receptor function and neurons obtained from said stem cells may be used as a genetic model for studying upper motor neurodegenerative disorders.

### METHODS FOR OBTAINING A CORTICOSPINAL UPPER MOTOR NEURON OF THE INVENTION

In one aspect, the invention relates to an *in vitro* method for obtaining a corticospinal upper motor neuron from a neural stem cell, wherein said method comprises contacting a neural stem cell under conditions adequate for increasing the signal mediated by the CB₁ cannabinoid receptor in said cells and under conditions to promote dorsalization of said cells.

The term "corticospinal upper motor neuron" or "UMN" refers to a motor neuron that originates in the brain cortex and carries motor information down to the final common pathway, that is, is not directly responsible for stimulating the target muscle. Corticospinal upper motor neurons control voluntary actions and lie mainly within layer V of the primary motor and somatosensory cortex. These neurons connect the brain to the appropriate level in the spinal cord, from which point nerve signals continue to the muscles by means of the lower motor neurons. A "corticospinal upper motor neuron" of the invention may be identified by the expression of Sox5, Ctip2, Uchl1, Tmem117/diap3 and/or Cdh22 markers at the transcript and protein level by real-time qPCR and immunofluorescence. Preferably, the markers for a corticospinal upper motor neuron are Sox5, Ctip2 and Uchl1, more preferably Ctip2 and Uchl1.

The term "corticospinal lower motor neuron" or "LMN" refers to a motor neuron that connects the brainstem and spinal cord to muscle fibers, bringing the nerve impulses from the upper motor neurons out to the muscles. A "corticospinal lower motor neuron" may be identified by the expression of islet1 (Karumbayaram et al., 2009), Hb9=MNx1, Lhx3 and/or ChAT (Hester et al., 2011) markers at the transcript and protein level by real-time qPCR and immunofluorescence.

The corticospinal upper motor neuron of the invention is obtained from differentiation of neural stem cells.

The term "stem cell" refers to a cell that, by successive divisions can give rise to specialized cells.

The term "neural stem cell", as used herein, refers to a self-renewing, multipotent cell that generates the main phenotypes of the nervous system. Neural stem cells primarily differentiate into neurons, astrocytes and oligodendrocytes. Neural stem cells are identified by the expression of nestin, musashi1, Sox2, CD133, vimentin and/or Notch markers. Neural stem cells may be isolated from various areas of the adult brain, including, without limitation, the adult striatal tissue, the subventricular zone (SVZ) of the brain, the cerebellum and non-neurogenic areas, such as the spinal cord.

Said neural stem cells may be derived from a pluripotent stem cell selected from a non-human embryonic stem cell (ES) and an induced pluripotent stem cell (iPS). Therefore, in an embodiment the neural stem cell is derived from a pluripotent stem cell selected from a non-human embryonic stem cell (ES) and an induced pluripotent stem cell (iPS).

The expression "derived", as used herein, refers to neural stem cells that originate from a pluripotent stem cell selected from a non-human embryonic stem cell (ES) and an induced pluripotent stem cell (iPS).

The term "pluripotent stem cell" refers to a stem cell that has the potential to differentiate into any of the three germ layers: endoderm (interior stomach lining, gastrointestinal tract, the lungs), mesoderm (muscle, bone, blood, urogenital), or ectoderm (epidermal tissues and nervous system). Pluripotent stem cells can give rise to any fetal or adult cell type but they cannot give rise to an entire organism. A "pluripotent stem cell" may be identified by the expression of one or more of the cell markers Klf4, Sox2, Oct4, cMyc, Nanog and SSEA1. A cell is considered a pluripotent stem cell when it is capable of generating cells from any of the three germ layers: endoderm, identified by the expression of alpha-fetoprotein; mesoderm (identified by the expression of desmin and/or alpha smooth muscle actin) and ectoderm (identified by the expression of beta-tubulin III = Tuj1 and/or E to N-cadherin). Assays to assess the pluripotentiality of a cell are known in the art ( 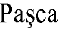 et al., 2011; Dimos et al., 2008). In the context of the present invention the term "pluripotent stem cell" includes non-human embryonic stem cells (ES) and induced pluripotent stem cells (iPS). In a preferred embodiment the pluripotent stem cell is selected from a non-human embryonic stem cell (ES) and an induced pluripotent stem cell (iPS).

The term "embryonic stem cell" or "ES" refers to a pluripotent stem cell that can be derived from the epiblast tissue of the inner cell mass (ICM) of a blastocyst or earlier morula stage embryos. For the purpose of the invention human embryonic stem cells are not encompassed by the term "embryonic stem cell". A "embryonic stem cell" is defined by the expression of several transcription factors and cell surface proteins including, without limitation, one or more of Oct4, Nanog, Sox2 and/or SSEA1 markers. Embryonic stem cells can be obtained from the inner cell mass of blastocysts by methods well-known by the person skilled in the art. Embryonic stem cells require different environments in order to maintain an undifferentiated state. For example, mouse ES cells are grown on a layer of gelatin as an extracellular matrix for support and require the presence of leukemia inhibitory factor (LIF). Without optimal culture conditions or genetic manipulation embryonic stem cells will rapidly differentiate. Culture conditions for ES cells are known in the art (Matise et al., 2000).

The term "induced pluripotent stem cell" or "iPS" refers to a pluripotent cell artificially derived from a non-pluripotent cell, typically an adult somatic cell, by inducing a forced expression of certain genes. A "induced pluripotent stem cell" is defined by the expression of several transcription factors including one or more of Klf4, Sox2, Oct4 and cMyc. iPS cells are typically derived by transfection of certain stem cell-associated genes into non-pluripotent cells, such as adult fibroblasts (Dimos et al., 2008). Transfection is typically achieved through viral vectors, such as retroviruses, and transfected genes include Oct-3/4 (Pou5fl) and Sox2. Additional genes include certain members of the Klf family (Klfl, Klf2, Klf4 and Klf5), the Myc family (c-myc, L-myc, N-myc), Nanog and LIN28 have been identified to increase the induction efficiency. After 3-4 weeks, small numbers of transfected cells begin to become morphologically and biochemically similar to pluripotent stem cells, and are typically isolated through morphological selection, doubling time, or through a reporter gene and antibiotic selection. Protocols for iPS culture are disclosed in Mochiduki and Okita, 2012.

Non-pluripotent cells that can be used to obtain iPS are, without limitation, fibroblasts, keratinocytes and adipocytes. These cells can be obtained from an adult being by methods well-known in the state of the art (Mochiduki and Okita, 2012).

The advantage of using iPS instead of ES is that iPS carry the genetics of the patient. Therefore, the corticospinal upper motor neurons obtained may be autologous, i.e. they come from cells from the same individual that is going to be treated with them, or whose individual condition is going to be studied / modeled.

The expression "contacting a neural stem cell under conditions adequate for increasing the signal mediated by the CB₁ cannabinoid receptor", as used herein, refers to the incubation of said cells in the presence of compounds or agents that produce an increase in the signal mediated by the CB₁ cannabinoid receptor. Examples of such agents may be, without limitation, CB₁ cannabinoid receptor agonists, agents that modify the neural stem cell to increase the expression of the CB₁ cannabinoid receptor above basal levels, agents that silence an endocannabinoid-degrading enzyme, inhibitors of a endocannabinoid-degrading enzyme and agents that increase the activity of an endocannabinoid-synthesizing enzyme above basal levels.

The term "CB₁ cannabinoid receptor" or "cannabinoid receptor type 1" refers to a G protein-coupled cannabinoid receptor primarily located in the brain. Said term encompasses the CB₁ cannabinoid receptor of any mammalian species, including but not being limited to domestic and farm animals (cows, horses, pigs, sheep, goats, dogs, cats or rodents), primates and humans. Preferably, the CB₁ cannabinoid receptor is human. In the present invention "human CB₁ cannabinoid receptor" is understood as the protein defined by the sequence of the Swiss-Prot database with accession number P21554 (release of October 3^{rd} 2012).

The CB₁ cannabinoid receptor activates multiple intracellular signal transduction pathways after it is activated by cannabinoids (Demuth and Molleman, 2006). Examples of such signal transduction pathways are the inhibition of the enzyme adenylate cyclase and the production of cAMP, the activation of inwardly rectifying potassium channels (=Kir or IRK), the regulation of adenylyl cyclase, MAP kinase, intracellular Ca(2+), and ion channels. In the context of the present invention, the expression "signal mediated by the CB₁ cannabinoid receptor" refers to the prevention of Satb2-mediated repression and the increase in Ctip2 promoter activity, thus generating Ctip2+ Satb2-neurons.

An increase in the signal mediated by the CB1 receptor, as used herein, refers to an increase in the number of Ctip2+ Satb2- cells with respect to the basal levels of at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. Quantification of Ctip2+ Satb2- cells is performed by densitometry after immunofluorescence as described in the experimental part of the present invention.

In a preferred embodiment, the increase in the signal mediated by the CB₁ cannabinoid receptor involves contacting said neural stem cell with a CB₁ cannabinoid receptor agonist and/or modifying said neural stem cell to increase the expression of the CB₁ cannabinoid receptor above basal levels.

The expression "CB₁ cannabinoid receptor agonist" refers to a compound capable of activating the CB₁ cannabinoid receptor including ligands, endocannabinoids produced by the mammalian body such as anandamide or 2-arachidonoylglycerol (2-AG), plant cannabinoids (such as THC, produced by the cannabis plant) and synthetic cannabinoids (such as HU-210 or dronabinol). Examples of CB₁ cannabinoid receptor agonists that can be used in this invention are, without limitation, endocannabinoids such as anandamide, 2-arachidonoylglycerol (2-AG) and N-arachidonoyl dopamine; synthetic cannabinoids such as arachidonyl-2'-chloroethylamide (ACEA), HU-210, JWH-073 and WIN 55,212-2. Cannabinoids acting as agonist for both the CB₁ and CB₂ receptors can also be used and include, without limitation, plant cannabinoids such as cannabinol, tetrahydrocannabinol (THC) and their metabolites 11-hydroxi-Δ⁹-tetrahydrocannabinol (11-OH-THC); synthetic cannabinoids such as levonantradol, dronabidol, AM-2201 (1-(5-fluoropentyl)-3-(1-naphthoyl)indole), JWH-018 (1-pentyl-3-(1-naphthoyl)indole) or AM-678; endocannabinoids such as 2-arachidonyl glyceryl ether (2-AGE, noladin ether), palmitoylethanolamide (PEA), anandamide or N-arachidonoylethanolamine or AEA and CP 55,940. In a preferred embodiment, the CB₁ cannabinoid receptor agonist is a synthetic agonist, preferably selected from the group consisting of HU-210 and arachydonyl-2'-chloroethylamide (ACEA); more preferably is HU-210. HU-210 is also called 1,1-dimethylheptyl- 11-hydroxytetrahydrocannabinol.

An assay to assess if a compound is a "CB₁ cannabinoid receptor agonist" suitable for the present invention consists on determining an increase in the number of Ctip2-only positive cells while decreasing the number of Satb2+ cells in a cortical organotypic culture as described in the experimental part of the present invention. An alternative way of assessing the activity of a CB₁ cannabinoid receptor agonist is to assay the ability of a CB₁ receptor antagonist such as SR141716A (Rinaldi-Carmona et al., 1995) or AM251 (Xi et al., 2006) to reverse the action produced by a CB₁ cannabinoid receptor agonist.

The expression "contacting" refers to the incubation of the stem cell in the presence of said compound.

Corticospinal motor neuron generation may be increased by genetic manipulation of the CB₁ cannabinoid receptor, i.e. by standard gene over-expression techniques. Therefore, in a preferred embodiment the increase in the signal mediated by the CB₁ cannabinoid receptor involves modifying said neural stem cell to increase the expression of the CB₁ cannabinoid receptor above basal levels.

The expression "modifying said neural stem cell" refers to the introduction in said stem cell of an expression vector encoding the CB₁ cannabinoid receptor by methods well-known by the skilled in the art.

The expression "basal levels" refers to the level of expression of a gene in a normal cell in conditions in which no manipulation of said cell has taken place.

The expression "expression levels" or its grammatical equivalent, as used herein, means a measurement of the amount of nucleic acid, e.g. RNA or mRNA, or protein of a gene in a cell, or alternatively, the level of activity of a gene or protein in said cell. Methods to assess such expression are well known in the art.

The value obtained for the expression of CB₁ cannabinoid receptor nucleic acid or protein levels in a modified cell is then compared with basal levels of CB₁ cannabinoid receptor in an unmodified cell. This allows detecting an increase in the expression of the CB₁ cannabinoid receptor above basal levels. An increase in the expression above basal levels, as used herein, refers to a change of expression of a given gene with respect to the expression basal levels of at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more.

In another embodiment, the increase in the signal mediated by the CB₁ cannabinoid receptor involves increasing in the cell the activity of an endocannabinoid-synthesizing enzyme above basal levels.

The term "endocannabinoid-synthesizing enzyme", as used herein, refers to an enzyme capable of synthesizing endocannabinoids. In a preferred embodiment the endocannabinoid-synthesizing enzyme is diacylglycerol lipase.

The term "diacylglycerol lipase", "DAG lipase", "DAGL" or "DGL" (E.C. 3.1.1) refers to an enzyme involved in the biosynthesis of the endocannabinoid 2-arachidonoylglycerol. It catalyzes the hydrolysis of diacylglycerol, releasing a free fatty acid and monoacylglycerol. The term "diacylglycerol lipase" encompasses the diacylglycerol lipase of any mammalian species, including but not being limited to domestic and farm animals (cows, horses, pigs, sheep, goats, dogs, cats or rodents), primates and humans. Preferably, the diacylglycerol lipase is human. Two human diacylglycerol lipase enzymes are known (DAGLA and DAGLB) encoded by different genes. In the present invention "human DAG lipase" is understood as the protein DAGLA defined by the sequence of the Swiss-Prot database with accession number Q9Y4D2 (release of October 31^{st} 2012) or the protein DAGLB defined by the sequence of the Swiss-Prot database with accession number Q8NCG7 (release of October 31^{st} 2012).

The activity of an endocannabinoid-synthesizing enzyme is assayed by measuring the quantity of endocannabinoid that has been synthesized by the enzyme. The assay depends on the specific enzyme and endocannabinoid involved.

The expression "basal levels", in the context of said embodiment, refers to the level of activity of an endocannabinoid-synthesizing enzyme in a normal cell in conditions in which no manipulation of said cell has taken place to increase the activity of said enzyme.

An increase in the activity of an endocannabinoid-synthesizing enzyme above basal levels, as used herein, refers to an increase in the levels of an endocannabinoid with respect to the basal levels of at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%.

In a preferred embodiment, the increase in the activity of an endocannabinoid-synthesizing enzyme is carried out by modifying said cell to increase the expression of an endocannabinoid-synthesizing enzyme.

The increase in the cell of the activity of an endocannabinoid-synthesizing enzyme may be achieved by the introduction in said cell of an expression vector encoding said enzyme by methods well-known by the skilled in the art.

The value obtained for the expression of an endocannabinoid-synthesizing enzyme nucleic acid or protein levels in a modified cell is compared with basal levels of the endocannabinoid-synthesizing enzyme in an unmodified cell to detect an increase in the expression of the endocannabinoid-synthesizing enzyme above basal levels. An increase in the expression above basal levels, as used herein, refers to a change of expression of a given gene with respect to the expression basal levels of at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more.

In another embodiment, the increase in the signal mediated by the CB₁ cannabinoid receptor involves decreasing in the cell the activity of an endocannabinoid-degrading enzyme. In a preferred embodiment, the decrease in the activity of an endocannabinoid-degrading enzyme is carried out by modifying said cell with an agent which silences said enzyme and/or contacting a neural stem cell with an inhibitor of said endocannabinoid-degrading enzyme.

The term "endocannabinoid-degrading enzyme", as used herein, refers to an enzyme capable of catabolizing endocannabinoids. In a preferred embodiment the endocannabinoid-degrading enzyme is selected from the group consisting of fatty acid amide hydrolase (FAAH) and monoacylglycerol lipase (MAGL).

The activity of an endocannabinoid-degrading enzyme is assayed by measuring the quantity of endocannabinoid that has been degraded by the enzyme. The assay depends on the specific enzyme and endocannabinoid involved.

The term "fatty acid amide hydrolase" or "FAAH" (EC 3.5.1.99) refers to a member of the serine hydrolase family of enzymes that is the principal catabolic enzyme for a class of bioactive lipids called the fatty acid amides (FAAs) that includes the endocannabinoid anandamide (N-arachidonoylethanolamine). The term "FAAH" encompasses the FAAH of any mammalian species, including but not being limited to domestic and farm animals (cows, horses, pigs, sheep, goats, dogs, cats or rodents), primates and humans. Preferably, the FAAH is human. Two human FAAH enzymes are known (FAAH-1 and FAAH-2) encoded by different genes. In the present invention "human FAAH" is understood as the protein FAAH-1 defined by the sequence of the Swiss-Prot database with accession number 000519 (release of October 3^{rd} 2012) or the protein FAAH-2 defined by the sequence of the Swiss-Prot database with accession number Q6GMR7 (release of October 3^{rd} 2012). Examples of selective inhibitors of FAAH useful in the present invention include, without limitation, URB597, urea based inhibitors such as PF-622, PF-750, PF-04457845, and PF3845. In a preferred embodiment the inhibitor of the FAAH enzyme is URB597 The inhibition of FAAH is typically assayed making use of a radiolabelled anandamide substrate (Nomura et al., 2008), which generates free labeled ethanolamine, although alternative LC-MS methods have also been described.

The term "monoacylglycerol lipase" or "MAGL" (EC 3.1.1.23) refers to an enzyme also known as MAG lipase that is a membrane-associated member of the serine hydrolase superfamily. It is a key enzyme in the hydrolysis of the endocannabinoid 2-arachidonoylglycerol. It converts monoacylglycerols to the free fatty acid and glycerol. The term "MAGL" encompasses the MAGL of any mammalian species, including but not being limited to domestic and farm animals (cows, horses, pigs, sheep, goats, dogs, cats or rodents), primates and humans. Preferably, the MAGL is human. In the present invention "human MAGL" is understood as the protein defined by the sequence of the Swiss-Prot database with accession number Q99685 (release of October 3^{rd} 2012). Examples of selective inhibitors of MAGL include, without limitation, JZL184. In a preferred embodiment the inhibitor of the MAGL enzyme is JZL184. MAGL activity is commonly detected by measuring free fatty acid release from a monoacylglycerol substrate using a liquid chromatography mass spectrometry system (Nomura et al., 2008) or the radiolabelled substrate 2-oleoyl-[³H]-glycerol.

A decrease in the activity of an endocannabinoid-degrading enzyme, as used herein, refers to an increase in the levels of an endocannabinoid with respect to the basal levels of at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%.
The generation of CSMN may be increased by decreasing the expression of an endocannabinoid-degrading enzyme by genetic manipulation. Therefore, in a preferred embodiment, the decrease in the activity of an endocannabinoid-degrading enzyme is carried out by modifying the neural stem cell with an agent which silences said enzyme.

The expression "modifying a neural stem cell" refers to the introduction in said stem cell of a shRNA, a siRNA, an antisense nucleic acid or a rybozime by methods well-known by the skilled in the art.

The expression "an agent which silences said enzyme" refers, as used herein, to a shRNA, a siRNA, an antisense nucleic acid or a rybozime capable of decreasing the expression of an endocannabinoid-degrading enzyme. An enzyme is silenced when its expression is decreased with respect to the expression basal levels by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%.

The expression "basal levels" refers to the level of expression of a gene in a normal cell in conditions in which no manipulation of said cell has taken place.

The expression "expression levels" or its grammatical equivalent, as used herein, means a measurement of the amount of nucleic acid, e.g. RNA or mRNA, or protein of a gene in a cell, or alternatively, the level of activity of a gene or protein in said cell.

As the person skilled in the art understands, the expression levels of FAAH or MAGL gene can be measured by determining the mRNA expression levels of said genes or by determining the protein levels encoded by said genes, i.e. the FAAH or MAGL protein.

In order to measure the mRNA levels of FAAH or MAGL genes, the cell may be treated to physically, mechanically or chemically disrupt cell structures, to release intracellular components into an aqueous or organic solution to prepare nucleic acids for further analysis. The nucleic acids are extracted from the sample by procedures known to the skilled person using commercially available reagents. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

Determination of the levels of FAAH or MAGL mRNA can be carried out by any method known in the art such as qPCR, northern blot, RNA dot blot, TaqMan®, tag based methods such as serial analysis of gene expression (SAGE) including variants such as LongSAGE and SuperSAGE, microarrays, nucleic acid sequence based amplification (NASBA), Fluorescence In Situ Hybridization, including variants such as Flow-FISH, qFiSH and double fusion fish (D-FISH) as described in WO2010030818, Femino et al. (Science, 1998, 280:585-590), Levsky et al. (Science, 2002, 297:836-840) or Raj et al. (PLoS Biology, 2006, 4:e309).

In order to normalize the values of mRNA expression among the different samples, it is possible to compare the expression levels of the mRNA of interest in the test samples with the expression of a control RNA. A "Control RNA" as used herein, relates to a RNA whose expression levels do not change or change only in limited amounts in stem cells. Preferably, the control RNA is mRNA derived from housekeeping genes and which code for proteins which are constitutively expressed and carry out essential cellular functions. Examples of housekeeping genes for use in the present invention include β-2-microglobulin, ubiquitin, 18-S ribosomal protein, cyclophilin, GAPDH, PSMB4, tubulin and β-actin.

The expression levels of FAAH or MAGL genes may also be measured by determining the protein levels encoded by said genes, i.e. the FAAH or MAGL proteins or variants thereof. Practically any conventional method can be used within the context of the present invention to quantify the levels of FAAH or MAGL proteins such as Western blot or immunohistochemistry.

The value obtained for the expression of FAAH or MAGL nucleic acid or protein levels in a modified cell is then compared with basal levels of FAAH or MAGL in an unmodified cell. This allows detecting a decrease in the expression of FAAH or MAGL under basal levels. A decrease in the expression under basal levels, as used herein, refers to a change of expression of a given gene with respect to the expression basal levels of at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%.
The decrease in the expression of an endocannabinoid-degrading enzyme may be achieved by a nucleic acid. The interference RNA technology may be used to interfere with the activity of the endocannabinoid-degrading enzyme. This approach may, for instance, utilize antisense nucleic acids or ribozymes that block translation of a specific mRNA, either by masking that mRNA with an antisense nucleic acid or cleaving it with a ribozyme. For a general discussion of antisense technology, see, e.g., Antisense DNA and RNA, (Cold Spring Harbor Laboratory, D. Melton, ed., 1988). If the inhibitor is a nucleic acid, it may be chemically synthesized, produced using *in vitro* transcription, etc.

Inhibition of genes encoding an endocannabinoid-degrading enzyme may also be useful. Such inhibition can be achieved by use of siRNAs (small interfering RNA), which is a class of double-stranded RNA molecules, 20-25 nucleotides in length with phosphorylated 5' ends and hydroxylated 3' ends with two overhanging nucleotides that interferes with the expression of specific genes with complementary nucleotide sequence. RNA interference (RNAi) technology prevents the expression of gene by using small RNA molecules such as small interfering RNAs (siRNAs). This technology in turn takes advantage of the fact that RNAi is a natural biological mechanism for silencing genes in most cells of many living organisms, from plants to insects to mammals (McManus et al., Nature Reviews Genetics, 2002, 3(10) p. 737). RNAi prevents a gene from producing a functional protein by ensuring that the molecule intermediate, the messenger RNA copy of the gene is destroyed. siRNAs can be used in a naked form and incorporated in a vector as shRNA, as described below. One can further make use of aptamers to specifically inhibit gene transcription of a gene encoding an endocannabinoid-degrading enzyme. See, for example, U.S. Patent 6,699,843. Aptamers useful in the present invention may be identified using the SELEX process. The methods of SELEX have been described in, for example, U. S. Patent Nos. 5,707,796, 5,763,177, 6,011,577, 5,580,737, 5,567,588, and 5,660,985.

The inhibition of an endocannabinoid-degrading enzyme may also be achieved by use of shRNA (short hairpin RNA), which is a sequence of RNA that makes a tight hairpin turn that can be used to silence target gene expression via RNA interference (RNAi). Expression of shRNA in cells is typically accomplished by delivery of plasmids or through viral vectors using polymerase II and polymerase III promoters.

siRNAs have been described in Brummelkamp et al., Science 296; 550-553,2002, Jaque et a1., Nature 418; 435-438, 2002, Elbashir S. M. et al. (2001) Nature, 411: 494-498, McCaffrey et al. (2002), Nature, 418: 38-39; Xia H. et al. (2002), Nat. Biotech. 20: 1006-1010, Novina et al. (2002), Nat. Med. 8: 681-686, and U.S. Application No. 20030198627.

In a preferred embodiment the agent that silences the endocannabinoid-degrading enzyme is a nucleic acid selected from the group consisting of a small interfering RNA (siRNA) and a short hairpin RNA (shRNA) specific for the mRNA of said enzyme.

The expression "specific for the mRNA of said enzyme", as used herein, refers to the capacity of the nucleic acids for binding specifically to their target sequence and not to other sequence. The specificity is validated by performing BLAST analysis, by the use of negative controls, and by determining changes in mRNA levels of unrelated genes.

CB₁ receptors may be activated by increasing endocannabinoid levels via inhibitors of their catabolizing enzymes. Therefore, in a preferred embodiment the decrease in the activity of an endocannabinoid-degrading enzyme is carried out by contacting a neural stem cell with an inhibitor of said endocannabinoid-degrading enzyme.

The term "inhibitor of an endocannabinoid-degrading enzyme" refers to a compound capable of inhibiting an enzyme that degrades endogenous agonists of cannabinoid receptors. Endocannabinoid-degrading enzymes included in the present invention are fatty acid amide hydrolase (FAAH) and monoacylglycerol lipase (MAGL). In a preferred embodiment the endocannabinoid-degrading enzyme is selected from the group consisting of fatty acid amide hydrolase (FAAH) and monoacylglycerol lipase (MAGL). In a preferred embodiment the inhibitor of MAGL is JZL184. In another preferred embodiment the inhibitor of FAAH is URB597.

Culture conditions for obtaining a corticospinal upper motor neuron from a neural stem cell are known in the art and involve the promotion of the dorsalization of the stem cell.

The expression "conditions to promote dorsalization", as used herein, refers to conditions suitable for forming dorsal neural cell types. In general the term dorsalization is applied, if in a developing system a change is introduced, which affects the subsequent differentiation of the system in such a way that the dorsal structures are produced at the expense of the ventral ones. Dorsalization denotes the change which leads to the differentiation of neural tissue, sense organs, crest cells and melanophores which are normally derived from the dorsal part of the ectoderm.

Examples of said culture conditions are defined default medium and a sonic hedgehog inhibitor (cyclopamine) (Gaspard, et al., 2008) or Smad inhibition (Shi, et al. 2012). Therefore, in a preferred embodiment the conditions to promote dorsalization involve contacting said neural stem cell with a dorsalizing agent selected from: a) a sonic hedgehog inhibitor; and b) a Smad inhibitor.

The term "dorsalizing agent" refers to a compound or a mixture of compounds capable of promoting dorsalization of the neural stem cell. Examples of said agents are, without limitation, a sonic hedgehog inhibitor or a Smad inhibitor.

The term "sonic hedgehog (Shh)" refers to the sonic hedgehog homolog and is one of the ligands in the mammalian signaling pathway family called hedgehog. It plays a key role in regulating vertebrate organogenesis and organization of the brain and it controls cell division of adult stem cells. Said term encompasses the sonic hedgehog (Shh) of any mammalian species, including but not being limited to domestic and farm animals (cows, horses, pigs, sheep, goats, dogs, cats or rodents), primates and humans. Preferably, the sonic hedgehog (Shh) is human. In the present invention "human sonic hedgehog (Shh)" is understood as the protein defined by the sequence of the Swiss-Prot database with accession number Q15465 (release of October 3^{rd} 2012).

The term "sonic hedgehog (Shh) inhibitor" refers to a compound capable of inhibiting the hedgehog signaling pathway by binding to Smoothened (Smo), which is the protein that is de-repressed when Shh binds to its receptor Patched (Ptch 1); by directly targeting Shh; or by inhibiting Shh signaling downstream of Smo. Examples of Shh inhibitors are, without limitation, adenosine 3',5'-cyclic monophosphate, N6,O2'-dibutyril- sodium salt, AY 9944, KAAD-cyclopamine, cyclopamine, forskolin, GANT58, GANT61, JK184, HPI-1, HPI-3, jervine, SANT-1, GDC-0449, robotnikinin, IPI-926. In a preferred embodiment the sonic hedgehog inhibitor is cyclopamine.
A Shh inhibitor is a compound that reduces the hedgehog signaling pathway with respect to the basal levels by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%.

The relative increase of Pax6 expressing cells and the relative reduction in the numbers of Olig2 expressing cells among all precursors would correspond to the dorsalization of the ventral spinal cord observed in the absence of Shh signaling (Ericson et al., 1997).

The action of a sonic hedhehog inhibitor may be assessed by determining the inhibition of the expression of Pax7 or Nkx2.2 (Briscoe et al., 1999; Wang et al., 2011) which are typical from ventral cells.

The term "Smad inhibitor", as used herein, refers to a compound capable of inhibiting the signal mediated by Smad (Small Mothers Against Decapentaplegic) protein. Such inhibitor interferes with a resulting target molecule or target compound or target process (i.e. reduces or eliminates or suppresses said target molecule, compound or process). Smads are intracellular proteins that transduce extracellular signals from transforming growth factor beta ligands to the nucleus where they activate downstream TGF-β gene transcription. Examples of Smad inhibitors are, without limitation, Noggin, Dorsomorphin, SB431542, LDN-193189, inhibitors that inhibit an anaplastic lymphoma kinase signaling pathway such as Lefty, Activin and TGF-beta. In a preferred embodiment a combination of two or more Smad inhibitors are used. In a more preferred embodiment the Smad inhibitor is selected from the group consisting of Noggin, SB431542 and a combination thereof.

A Smad inhibitor is a compound or mixture of compounds that reduces the signal mediated by Smad protein with respect to the basal levels by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%.

A Smad inhibitor useful for the present invention may be identified by detecting a decrease in the signal mediated by Smad protein in cells treated with the compound with respect to untreated cells.

### CORTICOSPINAL UPPER MOTOR NEURONS OF THE INVENTION

In another aspect, the disclosure relates to a corticospinal upper motor neuron obtainable by a method of the invention.

The term "corticospinal upper motor neuron" has been defined in the first aspect of the invention. The corticospinal upper motor neurons obtained by the method according to the invention are characterized by showing one or more of the following features:
- The expression of Sox5, Ctip2, Uchl1, Tmem117/diap3 and/or Cdh22 markers at the transcript and protein level by real-time qPCR and immunofluorescence. Preferably, the markers for a corticospinal upper motor neuron are Sox5, Ctip2 and Uchl1, more preferably Ctip2 and Uchl1.
- Their typical deep layer morphology (e.g. one apical long dendrite towards the pia and basal dendrites into deep layer 5 (Ideguchi et al., 2010; Gaspard et al., 2008; Eiraku et al., 2008).

The methods of the present invention allow obtaining a cortical culture enriched in UMN cells.

Another aspect of the disclosure is a cell population comprising corticospinal upper motor neurons obtainable by a method of the invention. Preferably, the cell population comprises at least 5%, at least 10%, at least 20%, at least 30%, at least 40%, at least 50%, at least 60%, at least 70%, at least 80%, at least 90%, at least 96%, 97%, 98% or 99% of corticospinal upper motor neurons of the invention; more preferably the cell population comprises at least 80% of corticospinal upper motor neurons of the invention.

### PHARMACEUTICAL COMPOSITIONS OF THE INVENTION

In another aspect, the disclosure relates to a pharmaceutical composition comprising a corticospinal upper motor neuron of the invention, or a cell population of the invention, and a pharmaceutically acceptable carrier.

The term "pharmaceutically acceptable" means approved by a regulatory agency of the Federal or a state government or listed in the U.S. Pharmacopeia, or European Pharmacopeia, or other generally recognized pharmacopeia for use in animals, and more particularly in humans. The term "carrier" refers to a diluent, adjuvant, excipient, or vehicle with which the therapeutic agent is administered. The composition, if desired, can also contain minor amounts of pH buffering agents. Examples of suitable pharmaceutical carriers are described in "Remington's Pharmaceutical Sciences" by E.W. Martin. Such compositions will contain a prophylactically or therapeutically effective amount of a corticospinal upper motor neuron or a cell population of corticospinal upper motor neurons of the invention preferably in purified form, together with a suitable amount of carrier so as to provide the form for proper administration to the subject. The formulation should suit the mode of administration. In a preferred embodiment, the pharmaceutical compositions are sterile and in suitable form for administration to a subject, preferably an animal subject, more preferably a mammalian subject, and most preferably a human subject.

The pharmaceutical composition of the disclosure may be in a variety of forms. These include, for example, solid, semi-solid, and liquid dosage forms, such as lyophilized preparations, liquids solutions or suspensions, injectable and infusible solutions, etc. The preferred form depends on the intended mode of administration and therapeutic application.

The administration of the cells or cell population of the disclosure or the pharmaceutical composition comprising same, to the subject in need thereof can be carried out by conventional means. In a particular embodiment, said cells or cell population is administered to the subject by a method which involves transferring the cells to the desired tissue, either *in vitro* or *in vivo,* to the animal tissue directly. The cells can be transferred to the desired tissue by any appropriate method, which generally will vary according to the tissue type. For example, cells can be seeded onto the desired site within the tissue to establish a population, etc. Cells can be transferred to sites *in vivo* using devices such as catheters, trocars, cannulae, stents (which can be seeded with the cells), etc.

The pharmaceutical composition of the disclosure can be used in a combination therapy. In a specific embodiment, the combination therapy is administered to a subject in need of treatment, such as a patient in need of repair or regeneration of a tissue. In an embodiment, the combination therapy is used in conjunction with other types of treatments to repair or regenerate neural tissue. In accordance with the above embodiment, the combination therapies of the invention can be used prior to, concurrently or subsequent to the administration of the cells of the invention.

### NON-THERAPEUTIC USES OF THE INVENTION

The corticospinal upper motor neuron or the cell population according to the disclosure is useful as a model to screen for active drugs. This allows minimizing the use of animal testing since compounds may be assayed *in vitro* with higher reliability. In addition, if the corticospinal upper motor neuron or the cell population of the invention is autologous, active drugs for a specific patient may be assayed.

Thus, another aspect of the disclosure relates to the use of a corticospinal upper motor neuron of the invention, or a cell population of the invention, for evaluating a biological, pharmacological and/or chemical product.

Compounds assayed may be biological, for example, without limitation, antibodies, proteins, nucleic acids, etc. Products with pharmacological activity may also be assayed as well as chemical products with non-pharmacological activity such as toxins.

### THERAPEUTIC USES OF THE INVENTION

The corticospinal upper motor neurons of the disclosure are useful for neural cell replacement therapies. Said therapies are based on the idea that neurological function lost to injury or neurodegeneration can be improved by introducing new cells that can form appropriate connections and replace the function of lost neurons.

Therefore, in another aspect the disclosure relates to a corticospinal upper motor neuron of the invention, a cell population of the invention or a pharmaceutical composition of the invention for use as a medicament.

In another aspect, the disclosure relates to a corticospinal upper motor neuron of the invention, a cell population of the invention or a pharmaceutical composition of the invention for use in the treatment of a motor neurodegenerative disorder. Alternatively, the invention relates to the use of a corticospinal upper motor neuron of the invention, a cell population of the invention or a pharmaceutical composition of the invention for the manufacture of a medicament for the treatment of a motor neurodegenerative disorder. Alternatively, the disclosure relates to a method for the treatment of a subject of a motor neurodegenerative disorder that comprises the administration to said subject of a corticospinal upper motor neuron, a cell population or a pharmaceutical composition of the invention.

The term "subject", as used herein, refers to all animals classified as mammals and includes, but is not restricted to, domestic and farm animals, primates and humans, e.g., human beings, non-human primates, cows, horses, pigs, sheep, goats, dogs, cats or rodents. Preferably, the subject is a male or female human of any age or race. In the context of the present invention, the subject is a subject suffering from a neurodegenerative disease or in risk of suffering a neurodegenerative disease.

The term "treat" or "treatment", as used herein, means achieving a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the subject, notwithstanding that the subject may still be afflicted with the underlying disorder. For prophylactic benefit, the compositions may be administered to a subject at risk of developing a particular disease, or to a subject reporting one or more of the physiological symptoms of a disease, even though a diagnosis of this disease may not have been made.

The expression "motor neurodegenerative disorder", as used herein, refers to a disease in which the nervous system progressively and irreversibly deteriorates and which selectively affect motor neurons (either upper motor neurons, lower motor neurons or both), the cells associated with voluntary and skeletal muscle movements. Examples of motor neurodegenerative disease, without limitation, are amyotrophic lateral sclerosis (ALS), primary lateral sclerosis (PLS), progressive muscular atrophy (PMA), progressive bulbar palsy (PBP), pseudobulbar palsy and spinal muscular atrophy. In a preferred embodiment the motor neurodegenerative disorder is amyotrophic lateral sclerosis (ALS). In another preferred embodiment the motor neurodegenerative disorder is primary lateral sclerosis (PLS).

### COMPOSITIONS OF THE INVENTION

The method of the invention involves incubating cells in the presence of a CB₁ cannabinoid receptor agonist or an inhibitor of an endocannabinoid-degrading enzyme and at least a dorsalizing agent. Therefore, it is the object of the present invention to provide compositions comprising said compounds.

Therefore, another aspect of the present invention is a composition comprising an agent capable of increasing the signal mediated by the CB₁ cannabinoid receptor in a neural stem cell and a further agent selected from: a) a sonic hedgehog inhibitor and b) a Smad inhibitor.

The term "composition", as used in this aspect, refers to a sterile liquid composition that contains: i) a CB₁ cannabinoid receptor agonist and/or an endocannabinoid-degrading enzyme and ii) a dorsalizing agent selected from a sonic hedgehog inhibitor and a Smad inhibitor. Said components may constitute a single formulation. Alternatively, the composition of the invention may comprise the components separately formulated, which are combined prior to use.

An agent capable of increasing the signal mediated by the CB₁ cannabinoid receptor in a neural stem cell is an agent capable of increasing the number of Ctip2+ Satb2- cells with respect to the basal levels by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. Quantification of Ctip2+ Satb2- cells is performed by densitometry after immunofluorescence as described in the experimental part of the present invention.

In a preferred embodiment the agent capable of increasing the signal mediated by the CB₁ cannabinoid receptor is a CB₁ cannabinoid receptor agonist and/or an inhibitor of an endocannabinoid-degrading enzyme.

The terms "sonic hedgehog inhibitor", "CB₁ cannabinoid receptor agonist", "inhibitor of an endocannabinoid-degrading enzyme", and "Smad inhibitor" have been defined previously in the context of the first aspect of the invention. All the embodiments applicable to the first aspect of the invention are also applicable to the composition of the invention.

In a preferred embodiment the sonic hedgehog inhibitor is cyclopamine. In another preferred embodiment the Smad inhibitor is selected from Noggin, SB431542 and a combination thereof. In another preferred embodiment the CB₁ cannabinoid receptor agonist is a synthetic agonist, preferably selected from the group consisting of HU-210 and arachidonyl-2'-chloroethylamine (ACEA), more preferably HU-210. In another preferred embodiment, the inhibitor of an endocannabinoid-degrading enzyme is selected from the group consisting of an inhibitor of fatty acid amide hydrolase (FAAH) and an inhibitor of monoacylglycerol lipase (MAGL), preferably selected from the group consisting of URB594 and JZL184.

### NEURAL STEM CELLS OF THE INVENTION, NEURONS OBTAINED BY IN VITRO DIFFERENTIATION OF SAID STEM CELLS AND USES THEREOF

The authors of the present invention have found that complete CB₁^{-/-} mice and glutamatergic neuron-specific Nex- CB₁^{-/-} mice have alterations in corticospinal motor neuron generation, thereby resulting in an impairment of skilled motor function in adult mice. Therefore, neural stem cells having a loss of the CB₁ cannabinoid receptor function and neurons obtained from said stem cells may be used as a model for studying motor neurodegenerative disorders (Maekawa et al., 2004).

In another aspect, the invention relates to an isolated neural stem cell having a loss of the CB₁ cannabinoid receptor function.

In the context of this aspect, the term "isolated" refers to a neural stem cell that is outside the animal or human body.

The expression "loss of the CB₁ cannabinoid receptor function" refers to a complete or partial decrease in the function of the CB₁ cannabinoid receptor.
A loss of the CB₁ cannabinoid receptor function, as used herein, refers to a decrease in the expression of the deep-layer neuronal markers Fezf2 and Ctip2 and the corticofugal neuronal marker Sox5 with respect to the basal levels of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. In addition, a loss of the CB₁ cannabinoid receptor function refers to an increase in the expression of the upper-layer transcription factor Satb2 with respect to the basal levels by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. Quantification of said markers is performed by densitometry after immunofluorescence, qPCR, western blot as described in the experimental part of the present invention.
The terms "neural stem cell" and "CB₁ cannabinoid receptor" have been defined previously in the context of the first aspect of the invention.

In another aspect, the invention relates to a population of neurons obtainable by *in vitro* differentiation from a neural stem cell having a loss of the CB₁ cannabinoid receptor function.

The population of neurons obtained by *in vitro* differentiation from a neural stem cell having a loss of the CB₁ cannabinoid receptor function may be identified by a decrease in the expression of Fezf2, Ctip2 and Sox5 markers and an increase in the expression of Satb2 marker with respect to a neuron obtained by *in vitro* differentiation from a neural stem cell having a functional CB₁ cannabinoid receptor. Said markers may be identified by real-time qPCR or immunofluorescence.

Culture conditions to differentiate a neuron from a neural stem cell having a loss of the CB₁ cannabinoid receptor function are typical culture conditions known in the art and involve the promotion of the dorsalization of the stem cell (Gaspardt, et al., 2008; Shi, et al., 2012).

In a preferred embodiment, the loss of the CB₁ cannabinoid receptor function is due to a partial or complete deletion or to a mutation in the CB₁ cannabinoid receptor.

The expression "partial deletion", as used herein, refers to the loss of at least 0.5%, 1%, 5%, 10%, 20%, 40%, 50%, 60%, 70%, 80%, 90% or 99% of the nucleotides forming the nucleotide sequence of a gene.

The expression "complete deletion", as used herein, refers to the loss of 100% of the nucleotides forming the nucleotide sequence of a gene.

The term "mutation", as used herein, refers to a change in a nucleic acid sequence. Said mutation include, but is not limited to, substitution (i.e. exchanging one or more nucleotides for others), inversion (i.e. a DNA segment inside a gene is inverted, to that end two 180° rotations are necessary, one for inverting the sequence and the other for maintaining the polarity of the DNA), translocation (i.e. a segment of a gene changes position to be in another different site of the same gene or in another site of the genome), and nucleotide insertions or deletions (i.e. the addition of one or more nucleotides (insertions or additions) or the loss of one or more nucleotides (deletions) having as a consequence changes in the reading frame, a reading error occurring during the translation ranging from the formation of non-functional proteins to the absence of said protein).

The generation of CB₁ cannabinoid receptors lacking its function is made by using genetic engineering techniques, for example by site-directed mutagenesis or gene deletion techniques well known in the art. See Brown T, "Gene Cloning" (Chapman & Hall, London, GB, 1995); Watson R, et al., "Recombinant DNA", 2nd Ed. (Scientific American Books, New York, NY, US, 1992); Alberts B, et al., "Molecular Biology of the Cell" (Garland Publishing Inc., New York, NY, US, 2008); Innis M, et al., Eds., "PCR Protocols. A Guide to Methods and Applications" (Academic Press Inc., San Diego, CA, US, 1990); Erlich H, Ed., "PCR Technology. Principles and Applications for DNA Amplification" (Stockton Press, New York, NY, US, 1989); Sambrook J, et al., "Molecular Cloning. A Laboratory Manual" (Cold Spring Harbor Laboratory Press, Cold Spring Harbor, NY, US, 1989); Bishop T, et al., "Nucleic Acid and Protein Sequence. A Practical Approach" (IRL Press, Oxford, GB, 1987); Reznikoff W, Ed., "Maximizing Gene Expression" (Butterworths Publishers, Stoneham, MA, US, 1987); Davis L, et al., "Basic Methods in Molecular Biology" (Elsevier Science Publishing Co., New York, NY, US, 1986), Schleef M, Ed., "Plasmid for Therapy and Vaccination" (Wiley-VCH Verlag GmbH, Weinheim, DE, 2001).

Alternatively, the neural stem cell having a loss of the CB₁ cannabinoid receptor function may be derived from iPS obtained from patients suffering from a motor neurodegenerative disorder.

Genetic deletions of CB₁ receptors cause alterations in corticospinal tract development. Therefore, in another aspect, the invention relates to the use of an isolated neural stem cell having a loss of the CB₁ cannabinoid receptor function or a population of neurons obtainable by *in vitro* differentiation from said neural stem cell as a model for studying a motor neurodegenerative disorder, preferably amyotrophic lateral sclerosis and primary lateral sclerosis.

n another aspect, the invention relates to the use of an isolated neural stem cell having a loss of the CB₁ cannabinoid receptor function or a population of neurons obtainable by *in vitro* differentiation from said neural stem cell for screening for compounds useful in the treatment of a motor neurodegenerative disorder, preferably selected from the group consisting of amyotrophic lateral sclerosis and primary lateral sclerosis.

Briefly, the present disclosure is aimed at:
[1]. An *in vitro* method for obtaining a corticospinal upper motor neuron from a neural stem cell, wherein said method comprises contacting a neural stem cell under conditions adequate for increasing the signal mediated by the CB₁ cannabinoid receptor in said cell and under conditions to promote dorsalization of said cell.
[2]. The method according to [1], wherein the increase in the signal mediated by the CB₁ cannabinoid receptor involves contacting said neural stem cell with a CB₁ cannabinoid receptor agonist and/or modifying said neural stem cell to increase the expression of the CB₁ cannabinoid receptor above basal levels and/or increasing in the cell the activity of an endocannabinoid-synthesizing enzyme above basal levels.
[3]. The method according to [2], wherein the CB₁ cannabinoid receptor agonist is a synthetic agonist.
[4]. The method according to [3], wherein the CB₁ cannabinoid receptor synthetic agonist is selected from the group consisting of HU-210 and arachidonyl-2'-chloroethylamide (ACEA).
[5]. The method according to [4], wherein the CB₁ cannabinoid receptor synthetic agonist is HU-210.
[6]. The method according to [1] to [5], wherein the increase in the signal mediated by the CB₁ cannabinoid receptor involves decreasing in the cell the activity of an endocannabinoid-degrading enzyme.
[7]. The method according to [6], wherein said decrease in the activity of an endocannabinoid-degrading enzyme is carried out by modifying said cell with an agent which silences said enzyme and/or contacting a neural stem cell with an inhibitor of said endocannabinoid-degrading enzyme.
[8]. The method according to [7], wherein the agent that silences the endocannabinoid-degrading enzyme is a nucleic acid selected from the group consisting of a small interfering RNA (siRNA) and a short hairpin RNA (shRNA) specific for the mRNA of said enzyme.
[9]. The method according to [6] to [8], wherein the endocannabinoid-degrading enzyme is selected from the group consisting of fatty acid amide hydrolase (FAAH) and monoacylglycerol lipase (MAGL).
[10]. The method according to [7] to [9], wherein the inhibitor of monoacylglycerol lipase is JZL184.
[11]. The method according to [7] to [9], wherein the inhibitor of fatty acid amide hydrolase is URB597.
[12]. The method according to [1] to [11], wherein the neural stem cell is derived from a pluripotent stem cell selected from a non-human embryonic stem cell (ES) and an induced pluripotent stem cell (iPS).
[13]. The method according to [1] to [12], wherein the conditions to promote dorsalization involve contacting said neural stem cell with a dorsalizing agent selected from: a) a sonic hedgehog inhibitor; and b) a Smad inhibitor.
[14]. The method according to [13], wherein the sonic hedgehog inhibitor is cyclopamine.
[15]. The method according to [13], wherein the Smad inhibitor is selected from the group consisting of Noggin, SB431542 and a combination thereof.
[16]. A corticospinal upper motor neuron obtainable by a method according to [1] to [15].
[17]. A cell population comprising corticospinal upper motor neurons according to [16].
[18]. A pharmaceutical composition comprising a corticospinal upper motor neuron according to [16] or a cell population according to [17], and a pharmaceutically acceptable carrier.
[19]. Use of a corticospinal upper motor neuron according to [16], or a cell population according to [17], for evaluating a biological, pharmacological and/or chemical product.
[20]. A corticospinal upper motor neuron according to [16], a cell population according to [17] or a pharmaceutical composition according to [18] for use as a medicament.
[21]. A corticospinal upper motor neuron according to [16], a cell population according to [17] or a pharmaceutical composition according to [18] for use in the treatment of a motor neurodegenerative disorder.
[22]. A corticospinal upper motor neuron, a cell population or a pharmaceutical composition for use according to [21], wherein the motor neurodegenerative disorder is selected from the group consisting of amyotrophic lateral sclerosis (ALS) and primary lateral sclerosis (PLS).
[23]. A composition comprising an agent capable of increasing the signal mediated by the CB₁ cannabinoid receptor in a neural stem cell and a further agent selected from: a) a sonic hedgehog inhibitor and b) a Smad inhibitor.
[24]. The composition according to [23], wherein the sonic hedgehog inhibitor is cyclopamine.
[25]. The composition according to [23] or [24], wherein the Smad inhibitor is selected from the group consisting of Noggin, SB431542 and a combination thereof.
[26]. The composition according to [23] to [25], wherein the agent capable of increasing the signal mediated by the CB₁ cannabinoid receptor is a CB₁ cannabinoid receptor agonist and/or an inhibitor of an endocannabinoid-degrading enzyme.
[27]. The composition according to [26], wherein the CB₁ cannabinoid receptor agonist is a synthetic agonist.
[28]. The composition according to [27], wherein the CB₁ cannabinoid receptor synthetic agonist is selected from the group consisting of HU-210 and arachidonyl-2'-chloroethylamide (ACEA).
[29]. The composition according to [28], wherein the CB₁ cannabinoid receptor synthetic agonist is HU-210.
[30]. The composition according to [26] to [29], wherein the inhibitor of an endocannabinoid-degrading enzyme is selected from the group consisting of an inhibitor of fatty acid amide hydrolase (FAAH) and an inhibitor of monoacylglycerol lipase (MAGL).
[31]. The compositon according to [30], wherein the inhibitor of monoacylglycerol lipase is JZL184.
[32]. The composition according to [30], wherein the inhibitor of fatty acid amide hydrolase is URB597.
[33]. An isolated neural stem cell having a loss of the CB₁ cannabinoid receptor function.
[34]. A population of neurons obtainable by *in vitro* differentiation from a neural stem cell having a loss of the CB₁ cannabinoid receptor function.
[35]. An isolated neural stem cell according to [33] or a population of neurons according to [34] wherein the loss of the CB₁ cannabinoid receptor function is due to a partial or complete deletion or to a mutation in the CB₁ cannabinoid receptor.
[36]. Use of an isolated neural stem cell or a population of neurons according to [33] to [35] as a model for studying a motor neurodegenerative disorder.
[37]. Use of an isolated neural stem cell or a population of neurons according to [33] to [35] for screening for compounds useful in the treatment of a motor neurodegenerative disorder.
[38]. Use according to [36] or [37] wherein the motor neurodegenerative disorder is selected from the group consisting of amyotrophic lateral sclerosis and primary lateral sclerosis.

The invention is described below by the following examples, which must be considered as merely illustrative and in no case limiting of the scope of the present invention.

### EXAMPLES

The following abbreviations are used in the experimental part:
2AG: 2-arachidonoylglycerol
BrdU, 5-bromo-2'-deoxyuridine
CB₁ receptor: cannabinoid type 1 receptor
COUP-TF: chicken ovoalbumin upstream promoter transcription factor
CP: cortical plate
CRE: Cre recombinase
CSMN: corticospinal motor neuron
Ctip2: COUP-TF interacting protein 2
DAPI: 4',6-diamidino-2-phenylindole
DiI: 1,1 '-diotadecyl-3,3,3 ',3 '-tetramethylindocarbocyanine
DsRed: *Discosoma sp.* red fluorescent protein
E16.5: embryonic day 16.5
eCB: endocannabinoid
F1: first filial generation
FAAH: fatty acid amide hydrolase
Fezf2: forebrain embryonic zinc finger like protein 2
GFP: green fluorescent protein
IgG: immunoglobulin G
IZ: intermediate zone
Ldb2: LIM domain-binding protein 2
MAR: matrix attachment region
P2: postnatal day 2
P8: postnatal day 8
P21: postnatal day 21
PBS: phosphate buffered saline
PKCγ: protein kinase C γ
Satb2: special AT-rich binding protein 2
Sox5: SRY (sex determining region Y)-box 5 transcription factor
Tbr1: T-box brain 1
Tbr2: T-box brain protein 2 or eomesodermin
Thy1: Thymidylate synthase 1
VZ/SVZ: ventricular/subventricular zone
WT: wild-type
YFP: yellow fluorescent protein

### MATERIALS AND METHODS

### Materials

The following materials were kindly donated: anti-CB₁ receptor antibody (K. Mackie, Indiana University, Bloomington, IN), FAAH^{-/-} mice (B. Cravatt, Scripps Institute, San Diego, CA), pCAG-DsRed (M. Nieto, National Center of Biotechnology, Madrid, Spain), pfosluc constructs with the MAR sequences A2 to A5 of the Ctip2 promoter and pMSCV-Satb2 expression vector (R. Grosschedl, Max Planck Institute of Immunobiology and Epigenetics, Freiburg, Germany) and HU-210 [(6a*R*,10a*R*)-3-(1,1_-dimethylheptyl)-6a,7,10,10a-tetrahydro-1-hydroxy-6,6-dimethyl-6*H*dibenzo[b,d]pyran-9-methanol] (R. Mechoulam, Hebrew University, Jerusalem, Israel). Rat monoclonal anti-BrdU, rabbit polyclonal anti-Ctip2, anti-Tbrl, anti-Tbr2, and mouse monoclonal anti-Satb2 antibodies were from Abcam (Cambridge, UK). Rabbit polyclonal anti-CB₁ antibody (Institute of Frontier Medical Sciences, Kyoto, Japan), mouse monoclonal anti-CRE (Covance), mouse monoclonal anti-GFP (Invitrogen) and chicken polyclonal anti-GFP (Millipore) antibodies were also used.

### Animals

Experimental designs and procedures were approved by the Complutense University Animal Research Committee in accordance with Directive 86/609/EU of the European Commission. All efforts were made to minimize the number of animals and their suffering throughout the experiments. Mice were maintained in standard conditions, keeping littermates grouped in breeding cages, at a constant temperature (20±2°C) on a 12-h light/dark cycle with food and water *ad libitum.* The generation and genotyping of CB₁^{-/-}, CB₁^{f/f,Nex-Cre}, CB₁^{f/f,Dlx5/6-Cre} and FAAH^{-/-} mice and their respective littermate controls have been reported previously and was performed accordingly (Cravatt et al., 2001; Marsicano et al., 2003; Monory et al., 2007; Massa et al., 2010). Thy1-eYFP (line H) mice were obtained from The Jackson Laboratory [B6.Cg-Tg(Thy1-YFP-H)2Jrs/J] and crossed with CB₁^{-/-} mice. The heterozygous F1 generation was crossed again with CB₁^{-/-} mice. Mouse tissues of either sex were obtained during timed mating as assessed by vaginal plug.

### Immunofluorescence and confocal microscopy

Coronal brain slices (10 µm) were processed as described previously (Mulder et al., 2008), and layers were identified by their discrete cell densities as visualized by Hoechst 33528 (Sigma) and β-III-tubulin counterstaining. After blockade with 5% goat serum, brain sections were incubated overnight at 4°C with the indicated primary antibodies. Confocal fluorescence images were obtained in a blinded manner by an independent observer, and all quantifications were obtained from a minimum of 6 sections from 1-in-10 series per mice. Immunofluorescence of cortical sections was performed along the rostral-to-caudal axis, and the quantifications were performed in the mediolateral area of rostro-medial sections that correspond to the motor/somatosensory cortex. Pyramidal layer specification was determined at E16.5, P2 and P8 in a 50 µm-wide cortical column divided into 10 equally-sized bins, from the ventricular surface to the marginal zone. At least 2 independent cortical columns were analyzed per section, and results were averaged (Figures 1, 2, 5 and 7). Positive cells for the corresponding markers were quantified and referred to the total cell number in the bin identified by Hoechst 33528.

### PKCγ immunohistochemistry

PKCy immunohistochemistry was performed in 100 µm-thick sagittal sections. After permeabilization, quenching of endogenous peroxidase activities and blockade with goat serum free-floating sections were incubated with rabbit anti-PKCy antibody (Santa Cruz Biotechnology) and processed according to instructions of the manufacturer with 1:500 goat biotinylated anti-rabbit IgG and with avidin-biotinylated peroxidase complex (ABC Standard Vectastain ABC kit; Vector Laboratories). Stained sections were mounted, and bright field images were captured from an Olympus BX51 upright microscope using an Olympus DP70 CCD camera.

### In situ hybridization

Coronal paraffin sections (10 µm) of embryonic days E12.5, 13.5, 14.5, and 16.5 heads were obtained, deparaffinized, and processed for *in situ* hybridization as described previously (Monory et al., 2007). Clim1 (National Center for Biotechnology Information reference sequence NM_ 010698.3) riboprobe for *in situ* hybridization was amplified with the following primers:
forward ACCCTCATTCCCCGTTATT (SEQ ID NO: 1), and
reverse TGGCTCTCCTACCACCATC (SEQ ID NO: 2).

### Real-time quantitative PCR

RNA was isolated using RNeasy Plus kit (Qiagen). cDNA was obtained with Transcriptor (Roche). Real-time quantitative PCR assays were performed using the FastStart master mix with Rox (Roche), and probes were obtained from the Universal Probe Library Set (Roche). Amplifications were run in a 7900 HT-Fast Real-Time PCR System (Applied Biosystems). Each value was adjusted by using 18S RNA and β-actin levels as reference.

### Ex vivo and in utero electroporation

*Ex vivo* electroporation experiments were performed at E13.5 or E14.5 as described previously (Mulder et al., 2008) by using pCAG-GFP and a pCAG-CB₁-GFP expression vectors, or shControl and shCB₁ were electroporated with mouse pGFP-V-RS (Origene) and dissociated cells were cultured as described below. In addition, CB₁^{f/f} cortices were electroporated with pCAG-GFP or pCAG-Cre-GFP (Addgene). In addition, *in utero* electroporations were performed at E13.5 or E14.5 with DsRed and pCAG-CB₁-GFP and (respectively) and analyzed at E16.5 (Fig. IF). *In utero* electroporation experiments with pCAG-CRE-GFP and pCAG control plasmids were performed from E12.5 to P0 in pregnant CB₁^{f/f} mice (Fig. 3D).

### Pyramidal and organotypical cortical cell cultures

Cortical neural progenitors were cultured from dissected cortices isolated at E13.5. Cells were mechanically dissociated and plated in polylysine- and laminin-coated dishes after *ex-utero* electroporation and dissection of the cortical electroporated area (fast green-positive). The neuronal phenotype was assessed after 7 days *in vitro* by quantification of Ctip2 and Satb2 expression in GFP⁺ cells from ≥10 randomly selected view fields/coverslip after Hoechst 33528 cell counterstaining in pharmacologically and genetically manipulated cells. In addition, pharmacological regulation experiments were performed in WT brain slices.

### Gene promoter activity assays

The neural stem cell line HiB5 (Renfranz et al., 1991) was used after transient transfection with 0.75 µg of the A4- or A3-MAR-pfosluc reporter of the Ctip2 promoter, 0.5 µg of pCAG-CB₁-GFP, 0.25 µg of pMSCV-Satb2, and 0.02 µg of renilla-derived luciferase as internal transfection control with Lipofectamine 2000 (Invitrogen). Transcriptional promoter-driven luciferase activity was performed using the Dual-Luciferase Reporter Promega (Madison, WI, USA) assay system and renilla-derived luciferase activity as internal transfection control in a Lumat LB9507 luminometer (Berthold Technologies). Control experiments were performed with an excess of Satb2 expression that was able to efficiently block the Ctip2-reporter activity (data not shown).

### Behavioral analyses

CB₁^{-/-} and Nex-CB₁^{-/-} mice and their respective wild-type littermates (WT and CB₁^{f/f}) were analyzed at 8 weeks of age with paired mean age among groups. Animals were always tested during the same light phase and acclimatized to the testing room for at least 30 min. All tests were video-recorded for subsequent analysis and double blind quantification. Mice were food-deprived the night before the testing day (mean body weight at the end of the test becoming 92.6 ± 2.2 % of the initial body weight). Skilled motor test and patch removal tasks were performed according to established protocols (Tomassy et al., 2010). Briefly, a plexiglas reaching box (20 cm long x 8 cm wide x 20 cm high), with a 1-cm-wide vertical slit in the front side of the box was used. Animals had to reach the palatable food pellet (20 mg dustless precision sucrose-flavored food pellets (Bio Serv., NJ, USA) from a shelf (4 cm wide x 8 cm long) in front of the vertical slit. Mice were habituated to the sucrose-flavored pellets for 3 consecutive days before the tests, which consisted of 3 phases: habituation, unskilled reaching, and skilled reaching. In the habituation phase (2 sessions), mice were placed in the testing cage and 10 pellets were scattered on the floor. The session finished when mice had eaten all the pellets or 10 min had passed. For the unskilled reaching test, food pellets were placed one by one on the shelf within the mouse's tongue-reaching distance. The test finished when 10 pellets were eaten or 4 min had passed. In the skilled reaching test, food pellets were placed one by one on the shelf 1.5 cm away from the slit, so that mice had to use their forelimbs to reach them. Pellet grasping and retrieval was scored as a success, and pellet displacement without retrieval was scored as an error. The test finished within 6 min. Results are represented as percentage of success [(total successes/total trials) x 100]. The absence of phenotypic alterations in CB₁^{-/-} and Nex-CB₁^{-/-} mice in the unskilled task was used as a control that CB₁ receptor ablation *per se* does not interfere with the test by influencing factors different from corticospinal function. Complementary analysis of motor impairment in CB₁^{-/-} mice was conducted by using the staircase reaching test (Campden Instruments Ltd., UK). This test allows measurement of coordinated paw-reaching in rodents. The system is formed by two stairs with 8 steps each on which 2 rewarding food pellets can be placed. The test consisted of three phases: training, unskilled reaching, and skilled reaching. Habituation to sucrose-flavored food pellets was performed for 3 consecutive days, and then mice were habituated to the apparatus for 2 days by placing some pellets along the central corridor and stairs. On 5 consecutive training days, both stairs were filled with 2 pellets per stair, and mice were challenged to reach pellets that had been placed exclusively in the stairs for 10 min. In 2 additional test sessions, animals were challenged to reach pellets placed in the 5 lowest steps, which need the use of a paw to be reached (note that the 3 upper steps can be reached with the tongue, and thus are not useful to assess paw skilled reaching ability). Finally, the patch removal test was conducted to measure skilled motor performance by assessing the ability of the mouse to remove a piece of adhesive patch placed in each hindpaw as described previously (Tomassy et al., 2010). Adhesive patches were placed in each hindpaw of the mouse, and the animal was placed in a normal housing cage and video-recorded. The test finished when both patches had been removed or 4 minutes had passed. The time of latency for the first nose contact with the patch was determined, thus providing a general assessment of the sensorial status. Skilled patch removal ability was measured by calculating the mean number of contacts until each adhesive patch had been removed. Results shown correspond to the average of two tests. Additional characterization of general motor activity, exploration, and coordination was performed in ActiTrack and RotaRod devices as described previously (Blazquez et al., 2011).

### DiI-labeling

Labeling of corticospinal tracts with the lipophilic carbocyanine dye DiI (1,1'-diotadecyl-3,3,3',3'-tetramethylindocarbocyanine; Invitrogen) was performed in the brains of Nex-CB₁^{-/-} and CB₁^{f/f} mice at P2. A single crystal of DiI was picked up on a fire-polished tip of a broken glass micropipette and inserted into the presumptive motor cortex. The DiI-containing brains were incubated in PBS 10 mM pH 7.4/0.1% sodium azide at 50°C in the dark for 4 weeks. Brains embedded in 3% agar were sectioned at 100 µm in the sagittal plane, counterstained with DAPI, and mounted with PBS. Axon projections were visualized using a Carl Zeiss Axiolmager M1 with 5X / 0.16 Zeiss objectives (magnification / numerical aperture). Confocal images were obtained using a Carl Zeiss 510 system with 10X / 0.45 numerical aperture (air) objective lens.

### Data analyses and statistics

Results shown represent the means ± S.E.M., and the number of experiments is indicated in every case. Statistical analysis was performed by one- or two-way ANOVA, as appropriate. A post hoc analysis was made by the Student-Neuman-Keuls test.

### EXAMPLE 1

### The CB₁ cannabinoid receptor regulates cortical layer neuron specification

Initially the impact of CB₁ receptor inactivation in cortical development was determined by analyzing cortical thickness of CB₁^{-/-} and wild-type (WT) littermates. CB₁^{-/-} mice showed enlarged ventricles at postnatal day 2 (P2), in agreement with the described alterations induced by *in utero* CB₁ receptor pharmacological blockade (Mulder et al., 2008). A reduction of total cortical thickness was evident at P2 in CB₁^{-/-}mice (total cortical thickness, 527 ± 26 µm in WT mice vs 475 ± 15 µm in CB₁^{-/-} mice; p<0.05), which was attributable to deep-layer but not upper-layer thickness reduction (upper- and deep-layer cortical thickness, 174 ± 26 and 352 ± 28 µm in WT mice vs 172 ± 5 and 302 ± 20 µm in CB₁^{-/-} mice; p<0.05 for deep-layer cortical thickness; n = 6 for each group). These results support a significant role of the CB₁ receptor in controlling progenitor population size and raise the question of whether CB₁ receptor signaling exerts a selective function in the differentiation of the neuronal populations of the different cortical layers. Because Tbr1 is an early expressed T-box transcription factor that promotes deep layer specification and corticothalamic neuron projections (Hevner et al., 2001; Han et al., 2011), postmitotic Tbr1⁺ neuroblasts were first analyzed along the developing cortex in CB₁ receptor-deficient mice. Quantification of Tbr1⁺ cells in equal-sized bins at E16.5 showed that these postmitotic cells accumulated abnormally in deep bins of the cortical plate of CB₁^{-/-} mice compared with WT littermates (Fig. 1A; immunofluorescence images not shown). Moreover, the distribution of neurons that express the upper-layer neuronal marker Satb2 was also affected and, in particular, Satb2⁺ cells were expanded in the lower bins 2-3 (Fig. 1B). Double marker analysis further showed that CB₁ receptor deletion increased the number of cells that coexpress Tbr1 and Satb2 (Fig. 1C). Satb2 is a well known repressor of Ctip2, a selective layer 5b marker of subcerebral projection neurons, and this repressive action of Satb2 promotes callosal projection-neuron identity (Alcamo et al., 2008). To investigate the impact of CB₁ deletion in the development of Satb2⁺ and Ctip2⁺ neuronal cell populations, Satb2⁺ cells in CB₁^{-/-} cortices at E16.5 were intermingled among Ctip2⁺ cells, and an increased number of cells coexpressing both markers were evident during CB₁ receptor inactivation (Fig. 1E). Satb2⁺ cell number in the total cortical column was significantly increased (Fig. 1D), and consequently Ctip2⁺ cells were reduced in CB₁^{-/-} mice compared with WT littermates (Fig. 1G).

Considering the altered expression of neuronal specification markers induced by genetic ablation of CB₁, gene expression analysis of selected transcription factors involved in the regulation of corticogenesis was performed. CB₁ receptor inactivation impaired the expression of determinants known to be involved in fate specification and development of deep- and upper-layer cortical neurons. Thus, CB₁ receptor deficiency decreased the expression of the deep-layer neuronal markers Fezf2 and Ctip2 (relative mRNA levels: 0.55 ± 0.07 versus 1.00 ± 0.15 in WT cortices and 0.47± 0.06 versus 1.00 ± 0.13 in WT cortices, p<0.05 and p<0.01, respectively; n = 4 for each group) (Arlotta et al., 2005; Chen et al., 2008) and the corticofugal neuronal marker Sox5 (relative mRNA levels: 0.50 ± 0.05 versus 1.00 ± 0.11 in WT cortices, p<0.05) (Lai et al., 2008). Conversely, levels of the upper-layer transcription factor Satb2 were upregulated in CB₁^{-/-} cortices (relative mRNA levels: 2.65 ± 0.15 versus 1.00 ± 0.23 in WT cortices, p<0.05). To validate the role of the CB₁ receptor in the correct expression of upper/deep-layer neuronal specification markers, a gain-of-function strategy aimed at rescuing CB₁ receptor expression in a CB₁^{-/-} background was performed by *in utero* electroporation of a pCAG-CB₁-GFP expression vector. Importantly, reexpression of the CB₁ receptor reduced the expansion of Satb2⁺ cells that occurred in deep bins of CB₁^{-/-} cortices, and thus reduced the number of GFP⁺Satb2⁺ cells (Fig. IF).

### EXAMPLE 2

### The CB₁ cannabinoid receptor controls deep-layer neuron specification through the regulation of Ctip2-Satb2 balance

The role of the CB₁ receptor in the regulation of deep-layer neuronal specification was further studied by analyzing the changes in the Ctip2-Satb2 axis in cortical organotypic cultures of E13.5 WT mice. Treatment with HU-210, a CB₁ receptor synthetic agonist, increased the number of Ctip2-only positive cells but decreased the number of Satb2⁺ cells (Fig. 2A-2B). The induction of neuronal differentiation to Ctip2⁺ cells by HU-210 and its inhibitory effect on Satb2⁺ cell differentiation were prevented by the CB₁ receptor-selective antagonist SR141716 [*N*-piperidino-5-(4-chlorophenyl)-1-(2,4-dichlorophenyl)-4-methyl-3-pyrazole carboxamide]. Because Satb2 negatively regulates Ctip2 expression by binding to MAR sequences at the Ctip2 promoter (Alcamo et al., 2008; Britanova et al., 2008), luciferase reporter assays for different MAR regions of the Ctip2 promoter were performed in the HiB5 neural stem cell line. HiB5 cells were transiently transfected with luciferase constructs under the control of the Satb2-binding sites A4 or A3 of the Ctip2 promoter together with expression vectors for CB₁ and Satb2. HU-210 treatment increased A4-fosluc activity (Fig. 2C), and this effect was prevented by CB₁ receptor blockade with SR141716. Likewise, HU-210 increased A3-fosluc activity (data not shown). Thus, CB₁ receptor activity prevents the repressive effect of Satb2 on the Ctip2 promoter, thereby increasing its activity.

To directly assess the role of the CB₁ receptor in deep-layer neuronal differentiation, E13.5 mouse cortices were electroporated when CSMN generation is maximal (Tomassy et al., 2010), with a GFP⁺ vector, and the CB₁ receptor was knocked-down or overexpressed with short hairpin RNA (shCB₁) and pCAG-CB₁, respectively. Cortical cultures were obtained by dissociation and allowed to differentiate for 7 days *in vitro.* Efficient manipulation of CB₁ receptor expression under these conditions was evaluated by CB₁ and GFP immunofluorescence (immunofluorescence images not shown). In addition, the effect of shCB₁ was validated by Western blot analysis (Fig. 3E) and real-time PCR. shCB₁ reduced CB₁ protein and mRNA levels when compared with shControl (relative CB₁ protein levels in shControl and shCB₁, 1.00 ± 0.13 and 0.59 ± 0.15, p < 0.01; relative CB₁ mRNA levels in shControl and shCB₁, 1.00 ± 0.23 and 0.46 ± 0.17, p<0.01). Immunofluorescence characterization of neuronal populations among the electroporated GFP⁺ population after differentiation revealed that CB₁ receptor downregulation decreased the generation of Ctip2-only-positive cells (Fig. 3A), whereas CB₁ receptor overexpression increased Ctip2⁺ cells (Fig. 3B). In addition, CB₁ ablation by *ex-utero* electroporation of pregnant E13.5 CB₁^{f/f} brains was conducted with a Cre recombinase expression vector. Using this strategy to achieve acute CB₁ receptor loss of function, we also observed reduced Ctip2⁺ differentiation of cortical cells when compared with CB₁^{f/f} cells (Fig. 3C). To further validate the results derived from *ex utero* CB₁ receptor manipulation, *in vivo in utero* electroporation was performed with the pCAG-Cre-GFP and pCAG-GFP plasmids, and pups were analyzed at P0. Noteworthy, *in utero* CB₁ receptor ablation decreased Ctip2⁺ neuronal differentiation when compared with control CB₁^{f/f} cortices (Fig. 3D). These results support that CB₁ receptor activity is required for the appropriate expression of the deep-layer neuronal determinant Ctip2.

### EXAMPLE 3

### The CB₁ cannabinoid receptor independently regulates progenitor proliferation and neuronal differentiation

Because CB₁ receptor loss of function is ensued by alterations in neuronal specification during cortical development (present study) and CB₁ receptor is also known to be expressed in progenitor cells, on which it drives VZ/SVZ progenitor proliferation in a cell-autonomous manner (Aguado et al., 2005; Mulder et al., 2008), a plausible hypothesis would be that CB₁ receptor-mediated regulation of neuronal specification was a direct consequence of its role on progenitor generation. To investigate the putative relationship between changes in cortical progenitor proliferation and neuronal specification, complete full CB₁^{-/-} mutant mice and glutamatergic-specific Nex-CB₁^{-/-} mutant mice were compared. In the latter mutants, CB₁ is selectively deleted in glutamatergic neurons of the dorsal telencephalon (Monory et al., 2006). Cre recombinase expression under the control of the Nex regulatory sequences was described to selectively target neurons of the developing cortex rather than cortical progenitors (Wu et al., 2005). However, the impact of conditional Nex-CB₁^{-/-} deletion during cortical development is unknown. Complete CB₁^{-/-} mice showed reduced progenitor proliferation in the developing cortex (Aguado et al., 2005), whereas no differences in progenitor cell proliferation were evident between CB₁^{f/f} and Nex-CB₁^{-/-}mice (Fig. 4A and 4B). Immunofluorescence analysis showed that Cre expression at embryonic day E14.5 occurred in postmitotic areas of the developing cortex beyond the basal edge of the SVZ, as identified by the expression pattern of its marker Tbr2 (data not shown). *In situ* hybridization experiments at different stages demonstrated that Nex-CB₁^{-/-} mice preserved CB₁ expression at early proliferative stages in VZ/SVZ cells and only postmitotic neuroblasts lost their expression at embryonic day E16.5 (data not shown). In agreement with the notion that the CB₁ receptor regulates neuronal differentiation independently of its actions on the progenitor cell pool, the observed increase in Satb2⁺ cells in CB₁^{-/-} was not attributable to its aberrant expression in apical or basal progenitor cells (data not shown). These findings demonstrated that the combined use of conditional Nex-CB₁^{-/-} and complete CB₁^{-/-} mice allows discriminating between CB₁ receptor actions in VZ/SVZ neural progenitor populations and CB₁ receptor-mediated regulation of differentiating postmitotic neuronal cells.

### EXAMPLE 4

### Deficient development of deep-layer Ctip2⁺ neurons in the absence of CB₁ cannabinoid receptor

To avoid the possible confounding interactions with impaired progenitor proliferation that occur in CB₁^{-/-} mice, the development of deep-layer Ctip2⁺ neurons in Nex-CB₁^{-/-} mice was investigated. The number of Ctip2⁺ cells at P2 along the cortical plate was reduced in Nex-CB₁^{-/-} mice compared with their WT littermates (Fig. 5A). Of importance, this effect was still evident at P8 (Fig. 5D). The direct regulatory action of CB₁ receptor in the specification of the pyramidal lineage was confirmed by analyzing Dlx5/6-CB₁^{-/-} mice, which lack CB₁ in GABAergic neurons of the forebrain (Monory et al., 2006). CB₁ receptor deletion in the GABAergic lineage did not interfere with the generation of Ctip2⁺ cells (Fig. 5B). Substantiating a putative regulatory action of the eCB tone in cortical layer specification, mice deficient in fatty acid amide hydrolase (FAAH), a major eCB-degrading enzyme, showed an increased number of Ctip2⁺ cells and, therefore, an opposite phenotype to Nex-CB₁^{-/-} mice (Fig. 5C). In addition, *in situ* hybridization for Clim1 (Ldb2), a transcription factor that labels subcerebral projection neurons of layer 5 (Azim et al., 2009), revealed a severe reduction of Clim1⁺ neurons in Nex-CB₁^{-/-} mice (data not shown). Furthermore, Western blot analysis of nuclear extracts from Nex-CB₁^{-/-} cortices showed reduced Ctip2 protein levels relative to Satb2 expression, whereas the opposite was observed in FAAH^{-/-} cortical extracts (Fig. 5E).

### EXAMPLE 5

### The CB₁ cannabinoid receptor regulates subcerebral projection neuron development

Aberrant corticofugal projections were found during CB₁ deletion. Immunofluorescence microscopy analysis of the L1 neural cell adhesion molecule revealed subcortical projection deficits in P2 CB₁^{-/-} and Nex-CB₁^{-/-} mice, but not in Dlx5/6-CB₁^{-/-} mice (data not shown). The corticostriatal boundary of Nex-CB₁^{-/-} mice showed altered axonal trajectories, and, in the intermediate zone (IZ), disorganized and enlarged fascicles were evident (data not shown). To investigate the role of the CB₁ receptor in subcerebral axonal projections, *in utero* electroporation experiments with pCAG-DsRed in CB₁^{-/-} mice at embryonic day E13.5 were performed, and DsRed⁺ projections at embryonic day E16.5 were analyzed. The corticospinal nature of DsRed-labeled axons was confirmed by the Ctip2⁺ immunoreactivity shown by DsRed⁺ somata. In wild-type (WT) embryos, labeled projecting axons expressed CB₁ receptor and showed straight trajectories, whereas profound alterations of navigating DsRed⁺ axons were observed at the IZ of CB₁-deficient mice (images not shown). To confirm more precisely the exact nature of these subcortical projection alterations, immunohistochemical analysis of protein kinase Cγ (PKCγ) was performed, because this protein is present in corticospinal tracts. Similar to the aberrant pattern of L1 immunofluorescence, abnormal axonal trajectories of PKCγ-labeled tracts in the corticostriatal junction were evident at P8 in Nex-CB₁^{-/-} mice compared with wild-type littermates. Although no major alterations of the corticospinal tract in the posterior hindbrain of Nex-CB₁^{-/-} were observed, these animals had aberrant CSMN projections as axons traverse the pons, those projections reaching the spinal cord in a notably less organized manner. Immunohistochemical images are not showed.

To unequivocally ascribe a role for the CB₁ receptor in the development of layer 5 subcerebral neurons, the inventors took advantage of Thy1-YFP-H mice, in which the expression of the fluorescent protein under the control of the neural-specific promoter of the Thy1 gene occurs selectively in layer 5 projection neurons, thus allowing the visualization of corticospinal tracts (Feng et al., 2000; Tomassy et al., 2010). CB₁^{-/-} and Thy1-YFP-H mice were crossed to study the specification and development of corticospinal neurons and axonal tracts in the absence of the CB₁ receptor. At P21, a significant reduction in fluorescently labeled somata was evident in cortical layer 5 (Fig. 6), whereas no changes were observed in the hippocampus (data not shown). CB₁ receptor deletion induced an aberrant phenotype of subcerebral projection neurons similar to that observed by PKC γ immunohistochemistry. Thus, misrouted axons were visible while traversing the internal capsule in the corticostriatal junction in CB₁^{-/-}:Thy1-YFP-H mice, axons that reached the pons were reduced, and the remaining ones showed fasciculation alterations. Finally, in agreement with a role of the CB₁ receptor in subcerebral projection neuron development, anterograde DiI tracing from the motor cortex of Nex-CB₁^{-/-} mice confirmed the existence of misrouted fibers branching off the tracts and deviating from their normal path at the corticostriatal junction. Immunohistochemical images are not shown.

### EXAMPLE 6

### The CB₁ cannabinoid receptor regulates corticospinal motor neuron function

The decreased Ctip2⁺ cell population and the disruption of subcerebral axonal projections observed in CB₁^{-/-} mice prompted the inventors to investigate whether these alterations of CSMN projections result in defective cortical motor function. Evaluation of the skilled pellet-reaching task, which is dependent on CSMN-mediated connectivity (Tomassy et al., 2010), revealed that adult CB₁^{-/-} mice had a remarkable impairment in fine motor function (Fig. 7A). The total number of trials performed during the skilled task was not significantly different between the two groups of mice, and unskilled motor activity was not affected either (Fig. 7C and 7D), thus indicating the selectivity of the skilled motor function deficits. Additional support for this selectivity was provided by the observation that the general motor activity, including total distance traveled, resting time and fast movements (ActiTrack test), as well as motor coordination (RotaRod test), did not differ between CB₁^{-/-} and WT mice (Fig. 8A). Moreover, and in concert with the neuroanatomical findings described above, the deficits in skilled motor activity observed in CB₁^{-/-} mice were recapitulated in Nex-CB₁^{-/-} mice (Fig. 7B).

Additional validation of the abnormal skilled-motor function phenotype found in CB₁^{-/-} animals was obtained by using the staircase test, which also reflects fine motor activity and is useful to evaluate motor impairment after cortical lesions (Brooks and Dunnett, 2009). CB₁^{-/-} and Nex-CB₁^{-/-} mice had a lower performance than their respective WT littermates in their ability to grasp the most difficult food pellets (steps 4-8) (Fig. 7E, 7F and 7G). Moreover, retrieval of non-challenging pellets in the staircase test (steps 1-3) was not different among genotypes (Fig. 7H), thus confirming the selective impairment of fine motor activity. Finally, the patch-removal task, which also evaluates sensorimotor function, was also used. Nex-CB₁^{-/-} mice were significantly less efficient than their WT littermates in removing a piece of adhesive tape from their hindpaws, as demonstrated by the higher number of contacts required for patch removal (Fig. 8B, left). This decreased performance of Nex-CB₁^{-/-} mice in patch removal reflected a fine motor function impairment rather than an altered perception of the patch, as indicated by the quantification of the latency time for the first attempt to remove the patch and the total time spent removing the patch, which were not significantly different between both genotypes (Fig. 8B, middle and right).

### CONCLUSIONS

The CB₁ receptor, by preventing Satb2-mediated repression, increased Ctip2 promoter activity and Ctip2⁺ neuron generation.

Unbalanced neurogenic fate determination found in complete CB₁^{-/-} mice and in glutamatergic neuron-specific Nex-CB₁^{-/-} mice induced overt alterations in corticospinal motor neuron generation and subcerebral connectivity, thereby resulting in an impairment of skilled motor function in adult mice.
Genetic deletion of CB₁ receptors in Thy1-YFP-H mice elicited alterations in corticospinal tract development.

The CB₁ receptor is required for the generation of deep-layer cortical neurons, specifically of corticospinal upper motor neurons, by coupling endocannabinoid signals from the neurogenic niche to the intrinsic proneurogenic Ctip2/Satb2 axis, thus dictating appropriate subcerebral projection neuron specification and corticospinal motor function in the adulthood.

### CITED LITERATURE

Aguado T, Monory K, Palazuelos J, Stella N, Cravatt B, Lutz B, Marsicano G, Kokaia Z, Guzman M, Galve-Roperh I (2005) The endocannabinoid system drives neural progenitor proliferation. FASEB J 19:1704-1706.
Alcamo EA, Chirivella L, Dautzenberg M, Dobreva G, Farinas I, Grosschedl R, McConnell SK (2008) Satb2 regulates callosal projection neuron identity in the developing cerebral cortex. Neuron 57:364-377.
Arlotta P, Molyneaux BJ, Chen J, Inoue J, Kominami R, Macklis JD (2005) Neuronal subtype-specific genes that control corticospinal motor neuron development in vivo. Neuron 45:207-221.
Azim E, Shnider SJ, Cederquist GY, Sohur US, Macklis JD (2009) Lmo4 and Clim1 progressively delineate cortical projection neuron subtypes during development. Cereb Cortex 19 Suppl 1:i62-69.
Blazquez C et al. (2011) Loss of striatal type 1 cannabinoid receptors is a key pathogenic factor in Huntington's disease. Brain 134:119-136.
Briscoe J, Sussel L, Serup P, Hartigan-O'Connor D, Jessell TM, Rubenstein JLR, Ericson J (1999) Homeobox gene Nkx2.2 and specification of neuronal identity by graded Sonic hedgehog signaling. Nature 398(6728): 622-7.
Britanova O, de Juan Romero C, Cheung A, Kwan KY, Schwark M, Gyorgy A, Vogel T, Akopov S, Mitkovski M, Agoston D, Sestan N, Molnar Z, Tarabykin V (2008) Satb2 is a postmitotic determinant for upper-layer neuron specification in the neocortex. Neuron 57:378-392.
Brooks SP, Dunnett SB (2009) Tests to assess motor phenotype in mice: a user's guide. Nat Rev Neurosci 10:519-529.
Bruijn, LI, Miller, TM, Cleveland, DW (2004) Unraveling the mechanisms involved in motor neuron degeneration in ALS. Annu Rev Neurosci 27: 723-749.
Chaddah MR, Dickie BG, Lyall D, Marshall CJ, Sykes JB, Bruijn LI (2011) Meeting report of the International Consortium of Stem Cell Networks' Workshop Towards Clinical Trials Using Stem Cells for Amyotrophic Lateral Sclerosis/Motor Neuron Disease. Amyotroph Lateral Scler 12(5): 315-317.
Chambers SM, Fasano CA, Papapetrou EP, Tomishima M, Sadelain M, Studer L (2009) Highly efficient neural conversion of human ES and iPS cells by dual inhibition of SMAD signaling. Nat Biotechnol 27(3):275-280.
Cravatt BF, Demarest K, Patricelli MP, Bracey MH, Giang DK, Martin BR, Lichtman AH (2001) Supersensitivity to anandamide and enhanced endogenous cannabinoid signaling in mice lacking fatty acid amide hydrolase. Proc Natl Acad Sci U S A 98:9371-9376.
Chen B, Wang SS, Hattox AM, Rayburn H, Nelson SB, McConnell SK (2008) The Fezf2-Ctip2 genetic pathway regulates the fate choice of subcortical projection neurons in the developing cerebral cortex. Proc Natl Acad Sci U S A 105:11382-11387.
Demuth DG, Molleman A (2006) Cannabinoid signaling. Life Sci 78(6):549-563.
Dimos JT, Rodolfa KT, Niakan KK, Weisenthal LM, Mitsumoto H, Chung W, Croft GF, Saphier G, Leibel R, Goland R, Wichterle H, Henderson CE, Eggan K (2008) Induced pluripotent stem cells generated from patients with ALS can be differentiated into motor neurons. Science 321(5893):1218-1221.
Eiraku M, Watanabe K, Matsuo-Takasaki M, Kawada M, Yonemura S, Matsumura M, Wataya T, Nishiyama A, Muguruma K, Sasai Y (2008) Self-organized formation of polarized cortical tissues from ESCs and its active manipulation by extrinsic signals. Cell Stem Cell 3(5):519-32.
Ericson J, Rashbass P, Schedl A, Brenner-Morton S, Kawakami A, van Heyningen V, Jessell TM, Briscoe J (1997) Pax6 controls progenitor cell identity and neuronal fate in response to graded. Cell. 90(1): 169-80.
Feng G, Mellor RH, Bernstein M, Keller-Peck C, Nguyen QT, Wallace M, Nerbonne JM, Lichtman JW, Sanes JR (2000) Imaging neuronal subsets in transgenic mice expressing multiple spectral variants of GFP. Neuron 28:41-51.
Gaspard, N, Bouschet, T, Hourez, R, Dimidschstein, J, et al. (2008) An intrinsic mechanism of corticogenesis from embryonic stem cells. Nature 455(7211):351-357.
Han W, Kwan KY, Shim S, Lam MM, Shin Y, Xu X, Zhu Y, Li M, Sestan N (2011) TBR1 directly represses Fezf2 to control the laminar origin and development of the corticospinal tract. Proc Natl Acad Sci U S A 108:3041-3046.
Hester ME, Murtha MJ, Song S, Rao M, Miranda CJ, Meyer K, Tian J, Boulting G, Schaffer DV, Zhu MX, Pfaff SL, Gage FH, Kaspar BK (2011) Rapid and efficient generation of functional motor neurons from human pluripotent stem cells using gene delivered transcription factor codes. Mol Ther 19(10):1905-1912.
Hevner RF, Shi L, Justice N, Hsueh Y, Sheng M, Smiga S, Bulfone A, Goffinet AM, Campagnoni AT, Rubenstein JL (2001) Tbr1 regulates differentiation of the preplate and layer 6. Neuron 29:353-366.
Ideguchi M, Palmer TD, Recht LD, Weimann JM (2010) Murine embryonic stem cell-derived pyramidal neurons integrate into the cerebral cortex and appropriately project axons to subcortical targets. J Neurosci 30(3):894-904.
Karumbayaram S, Novitch BG, Patterson M, Umbach JA, Richter L, Lindgren A, Conway AE, Clark AT, Goldman SA, Plath K, Wiedau-Pazos M, Kornblum HI, Lowry WE (2009) Directed differentiation of human-induced pluripotent stem cells generates active motor neurons. Stem cells 27(4):806-811.
Lai T, Jabaudon D, Molyneaux BJ, Azim E, Arlotta P, Menezes JR, Macklis JD (2008) SOX5 controls the sequential generation of distinct corticofugal neuron subtypes. Neuron 57:232-247.
Maekawa S, Al-Sarraj S, Kibble M, Landau S, Parnavelas J, Cotter D, Everall I, Leigh PN (2004) Cortical selective vulnerability in motor neuron disease: a morphometric sstudy. Brain 127:1237-1251.
Mangelsdorf DJ, Umesono K, Kliewer SA, Borgmeyer U, Ong ES, Evans RM (1991) A direct repeat in the cellular retinol-binding protein type II gene confers differential regulation by RXR and RAR. Cell 66(3):555-61.
Marsicano G, Goodenough S, Monory K, Hermann H, Eder M, Cannich A, Azad SC, Cascio MG, Gutierrez SO, van der Stelt M, Lopez-Rodriguez ML, Casanova E, Schutz G, Zieglgansberger W, Di Marzo V, Behl C, Lutz B (2003) CB1 cannabinoid receptors and on-demand defense against excitotoxicity. Science 302:84-88.
Massa F, Mancini G, Schmidt H, Steindel F, Mackie K, Angioni C, Oliet SH, Geisslinger G, Lutz B (2010) Alterations in the hippocampal endocannabinoid system in diet-induced obese mice. J Neurosci 30:6273-6281.
Matise MP, Auerbach W, Joyner AL (2000) Production of targeted embryonic stem cell clones in: Joyner A.L. (ed.)-Gene Targeting, a practical approach, 2nd edition. The Practical Approach Series, Oxford University Press.
Mochiduki Y, Okita K (2012) Methods for iPS cell generation for basic research and clinical applications. Biotechnol J, 7(6):789-97.
Monory K, Blaudzun H, Massa F, Kaiser N, Lemberger T, Schutz G, Wotjak CT, Lutz B, Marsicano G (2007) Genetic dissection of behavioural and autonomic effects of Delta(9)-tetrahydrocannabinol in mice. PLoS Biol 5:e269.
Monory K et al. (2006) The endocannabinoid system controls key epileptogenic circuits in the hippocampus. Neuron 51:455-466.
Mulder J, Aguado T, Keimpema E, Barabas K, Ballester Rosado CJ, Nguyen L, Monory K, Marsicano G, Di Marzo V, Hurd YL, Guillemot F, Mackie K, Lutz B, Guzman M, Lu HC, Galve-Roperh I, Harkany T (2008) Endocannabinoid signaling controls pyramidal cell specification and long-range axon patterning. Proc Natl Acad Sci U S A 105:8760-8765.
Nomura DK, Blankman JL, Simon GM, Fujioka K, Issa RS, Ward AM, Cravatt BF, Casida JE (2008) Activation of the endocannabinoid system by organophosphorus nerve agents. Nat Chem Biol 4(6):373-8.
Ozdinler PH, Benn S, Yamamoto TH, Guzel M, et al. (2011) Corticospinal motor neurons and related subcerebral projection neurons undergo early and specific neurodegeneration in hSOD1G(3)A transgenic ALS mice. J Neurosci, 31(11): 4166-4177.
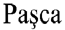 SP, Portmann T, Voineagu I, Yazawa M, Shcheglovitov A, 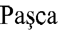 AM, Cord B, Palmer TD, Chikahisa S, Nishino S, Bernstein JA, Hallmayer J, Geschwind DH, Dolmetsch RE (2011) Using iPSC-derived neurons to uncover cellular phenotypes associated with Timothy syndrome. Nat Med, 17(22):1657-62.
Renfranz PJ, Cunningham MG, McKay RD (1991) Region-specific differentiation of the hippocampal stem cell line HiB5 upon implantation into the developing mammalian brain. Cell, 66(4):713-29.
Rinaldi-Carmona M, Barth F, Héaulme M, Alonso R, Shire D, Congy C, Soubrié P, Brelière JC, Le Fur G (1995) Biochemical and pharmacological characterisation of SR141716A, the first potent and selective brain cannabinoid receptor antagonist. Life Sci, 56(23-24):1941-7.
Sach M, Winkler G, Glauche V, Liepert J, Heimbach B, Koch MA, Büchel C, Weiller C (2004) Diffusion tensor MRI of early upper motor neuron involvement in amyotrophic lateral sclerosis. Brain, 127: 340-350.
Shi, Y, Kirwan, P, Smith, J, Robinson, HP, et al. (2012) Human cerebral cortex development from pluripotent stem cells to functional excitatory synapses. Nat Neurosci, 15(3): 477-486, S471.
Tomassy GS, De Leonibus E, Jabaudon D, Lodato S, Alfano C, Mele A, Macklis JD, Studer M (2010) Area-specific temporal control of corticospinal motor neuron differentiation by COUP-TFI. Proc Natl Acad Sci U S A 107:3576-3581.
Wang H, Lei Q, Oosterveen T, Ericson J, Matise MP (2011) Tcf/Lef repressors differentially regulate Shh-Gli target gene activation thresholds to generate progenitor patterning in the developing CNS. Development, 138(17):3711-21.
Wichterle H, Lieberam I, Porter JA, Jessell TM (2002) Directed differentiation of embryonic stem cells into motor neurons. Cell 110(3):385-97.
Wu SX, Goebbels S, Nakamura K, Kometani K, Minato N, Kaneko T, Nave KA, Tamamaki N (2005) Pyramidal neurons of upper cortical layers generated by NEX-positive progenitor cells in the subventricular zone. Proc Natl Acad Sci USA 102:17172-17177.
Xi ZX, Gilbert JG, Peng XQ, Pak AC, Li X, Gardner EL (2006) Cannabinoid CB1 receptor antagonist AM251 inhibits cocaine-primed relapse in rats: role of glutamate in the nucleus accumbens. J Neurosci 26(33):8531-6.

## Claims

1. An *in vitro* method for obtaining a corticospinal upper motor neuron from a neural stem cell, wherein said method comprises contacting a neural stem cell under conditions adequate for increasing the signal mediated by the CB₁ cannabinoid receptor in said cell and under conditions to promote dorsalization of said cell and wherein conditions adequate for increasing the signal mediated by the CB₁ cannabinoid receptor involve an increase in the number of Ctip2+ Satb2- cells with respect to the basal levels of at least 5% and involve:
(i) contacting said neural stem cell with a CB₁ cannabinoid receptor agonist selected from the group consisting of anandamide, 2-arachidonoylglycerol (2-AG), N-arachidonoyl dopamine, arachidonyl-2'-chloroethylamide (ACEA), HU-210, JWH-073, WIN 55,212-2, cannabinol, tetrahydrocannabinol (THC), 11-hydroxy-Δ⁹-tetrahydrocannabinol (11-OH-THC), levonantradol, dronabinol, AM-2201 (1-(5-fluoropentyl)-3-(1-naphthoyl)indole), JWH-018 (1-pentyl-3-(1-naphthoyl)indole), AM-678, 2-arachidonyl glyceryl ether (2-AGE, noladin ether), palmitoylethanolamide (PEA) and CP 55,940; and/or
(ii) introducing in said neural stem cell an expression vector encoding the CB₁ cannabinoid receptor to increase the expression of the CB₁ cannabinoid receptor above basal levels, and/or
(iii) introducing in said neural stem cell an expression vector encoding an endocannabinoid-synthesizing enzyme to increase in the cell the activity of an endocannabinoid-synthesizing enzyme above basal levels, and/or
(iv) introducing in said neural stem cell an agent which silences an endocannabinoid-degrading enzyme selected from the group consisting of a shRNA, a siRNA, an antisense nucleic acid and a ribozyme and/or contacting said neural stem cell with an inhibitor of said endocannabinoid-degrading enzyme,
and wherein conditions to promote dorsalization of said cell involve contacting said neural stem cell with a dorsalizing agent selected from:
a) a sonic hedgehog inhibitor selected from the group consisting of adenosine 3',5'-cyclic monophosphate, N6,O2'-dibutyryl- sodium salt, AY 9944, KAAD-cyclopamine, cyclopamine, forskolin, GANT58, GANT61, JK184, HPI-1, HPI-3, jervine, SANT-1, GDC-0449, robotnikinin and IPI-926; and
b) a Smad inhibitor selected from the group consisting of Noggin, Dorsomorphin, SB431542, LDN-193189, Lefty, Activin, TGF-beta and combinations thereof.

2. The method according to claim 1, wherein the CB₁ cannabinoid receptor agonist is selected from the group consisting of HU-210 and arachidonyl-2'-chloroethylamide (ACEA).

3. The method according to any of claims 1 or 2, wherein the endocannabinoid-synthesizing enzyme is diacylglycerol lipase.

4. The method according to any of claims 1 to 3, wherein the endocannabinoid-degrading enzyme is selected from the group consisting of fatty acid amide hydrolase (FAAH) and monoacylglycerol lipase (MAGL).

5. The method according to any of claims 1 to 4, wherein the inhibitor of the endocannabinoid-degrading enzyme is selected from the group consisting of URB597, PF-622, PF-750, PF-04457845, PF3845 and JZL184.

6. The method according to claim 5, wherein the inhibitor of the endocannabinoid-degrading enzyme is selected from the group consisting of JZL184 and URB597.

7. The method according to any of claims 1 to 5, wherein the sonic hedgehog inhibitor is cyclopamine and/or the Smad inhibitor is selected from the group consisting of Noggin, SB431542 and a combination thereof.

8. A composition comprising an agent capable of increasing the signal mediated by the CB₁ cannabinoid receptor in a neural stem cell, wherein said increase involves an increase in the number of Ctip2+ Satb2- cells with respect to the basal levels of at least 5%, said agent selected from the group consisting of:
(i) a CB₁ cannabinoid receptor agonist selected from the group consisting of anandamide, 2-arachidonoylglycerol (2-AG), N-arachidonoyl dopamine, arachidonyl-2'-chloroethylamide (ACEA), HU-210, JWH-073, WIN 55,212-2, cannabinol, tetrahydrocannabinol (THC), 11-hydroxy-Δ⁹-tetrahydrocannabinol (11-OH-THC), levonantradol, dronabinol, AM-2201 (1-(5-fluoropentyl)-3-(1-naphthoyl)indole), JWH-018 (1-pentyl-3-(1-naphthoyl)indole), AM-678, 2-arachidonyl glyceryl ether (2-AGE, noladin ether), palmitoylethanolamide (PEA) and CP 55,940; and/or
(ii) an inhibitor of an endocannabinoid-degrading enzyme
and a dorsalizing agent selected from:
a) a sonic hedgehog inhibitor selected from the group consisting of adenosine 3',5'-cyclic monophosphate, N6,O2'-dibutyryl- sodium salt, AY 9944, KAAD-cyclopamine, cyclopamine, forskolin, GANT58, GANT61, JK184, HPI-1, HPI-3, jervine, SANT-1, GDC-0449, robotnikinin and IPI-926; and
b) a Smad inhibitor selected from the group consisting of Noggin, Dorsomorphin, SB431542, LDN-193189, Lefty, Activin, TGF-beta and combinations thereof.

9. The composition according to claim 8, wherein the CB₁ cannabinoid receptor agonist is selected from the group consisting of HU-210 and arachidonyl-2'-chloroethylamide (ACEA).

10. The composition according to any of claims 8 and 9, wherein the inhibitor of an endocannabinoid-degrading enzyme is selected from the group consisting of URB597, PF-622, PF-750, PF-04457845, PF3845 and JZL184.

11. The composition according to claim 10, wherein the inhibitor of an endocannabinoid-degrading enzyme is selected from the group consisting of JZL184 and URB597.

12. The composition according to any of claims 8 to 11, wherein the sonic hedgehog inhibitor is cyclopamine and/or the Smad inhibitor is selected from the group consisting of Noggin, SB431542 and a combination thereof.

13. In vitro use of a composition according to any one of claims 8 to 12 for obtaining a corticospinal upper motor neuron from a neural stem cell.

## Patentansprüche

1. *In vitro*-Verfahren zum Erhalten eines kortikospinalen oberen Motorneurons aus einer neuralen Stammzelle, wobei das Verfahren das Inkontaktbringen einer neuralen Stammzelle unter Bedingungen umfasst, die zur Erhöhung des Signals, das von dem CB₁-Cannabinoidrezeptor vermittelt wird, in der Zelle geeignet sind und unter Bedingungen, die die Dorsalisierung der Zelle zu fördern, und wobei die Bedingungen, die zur Erhöhung des Signals, das von dem CB₁-Cannabinoidrezeptor vermittelt wird, geeignet sind, einen Anstieg der Anzahl von Ctip2(+)Satb2(-)-Zellen in Bezug auf die Basalspiegel von mindestens 5% zur Folge haben und beinhalten:
(i) Inkontaktbringen der neuralen Stammzelle mit einem CB₁-Cannabinoidrezeptor-Agonisten, der ausgewählt ist aus der Gruppe bestehend aus Anandamid, 2-Arachidonylglycerol (2-AG), N-Arachidonyldopamin, Arachidonyl-2'-chlorethylamid (ACEA), HU-210, JWH-073, WIN 55,212-2, Cannabinol, Tetrahydrocannabinol (THC), 11-Hydroxy-Δ⁹-tetrahydrocannabinol (11-OH-THC), Levonantradol, Dronabinol, AM-2201 (1-(5-Fluorpentyl)-3-(1-naphthoyl)indol), JWH-018 (1-Pentyl-3-(1-naphthoyl)indol), AM-678, 2-Arachidonylglycerylether (2-AGE, Noladin-Ether), Palmitoylethanolamid (PEA) und CP 55,940; und/oder
(ii) Einführen eines Expressionsvektors, der den CB₁-Cannabinoidrezeptor codiert, in die neurale Stammzelle, um die Expression des CB₁-Cannabinoidrezeptors über die Basalspiegel zu erhöhen, und/oder
(iii) Einführen eines Expressionsvektors, der ein Endocannabinoidsynthetisierendes Enzym codiert, in die neurale Stammzelle, um in der Zelle die Aktivität eines Endocannabinoid-synthetisierenden Enzyms über die Basalspiegel zu erhöhen, und/oder
(iv) Einführen eines Agens, das ein Endocannabinoid-abbauendes Enzym ausschaltet, das ausgewählt ist aus der Gruppe bestehend aus einer shRNA, einer siRNA, einer Antisense-Nucleinsäure und einem Ribozym, in die neurale Stammzelle und/oder Inkontaktbringen der neuralen Stammzelle mit einem Inhibitor des Endocannabinoid-abbauenden Enzyms,
und wobei die Bedingungen, die die Dorsalisierung der Zelle fördern, das Inkontaktbringen der neuralen Stammzelle mit einem Dorsalisierungsagens beinhalten, das ausgewählt ist aus:
a) einem Sonic-Hedgehog-Inhibitor, der ausgewählt ist aus der Gruppe bestehend aus cyclischem Adenosin-3',5'-monophosphat, N6,O2'-Dibutyryl-Natriumsalz, AY 9944, KAAD-Cyclopamin, Cyclopamin, Forskolin, GANT58, GANT61, JK184, HPI-1, HPI-3, Jervin, SANT-1, GDC-0449, Robotnikinin und IPI-926; und
b) einem Smad-Inhibitor, der ausgewählt ist aus der Gruppe bestehend aus Noggin, Dorsomorphin, SB431542, LDN-193189, Lefty, Activin, TGF-beta und Kombinationen davon.

2. Verfahren nach Anspruch 1, wobei der CB₁-Cannabinoidrezeptor-Agonist ausgewählt ist aus der Gruppe bestehend aus HU-210 und Arachidonyl-2'-chlorethylamid (ACEA).

3. Verfahren nach einem der Ansprüche 1 oder 2, wobei das Endocannabinoid-synthetisierende Enzym Diacylglycerol-Lipase ist.

4. Verfahren nach einem der Ansprüche 1 bis 3, wobei das Endocannabinoid-abbauende Enzym ausgewählt ist aus der Gruppe bestehend aus Fettsäureamid-Hydrolase (FAAH) und Monoacylglycerin-Lipase (MAGL).

5. Verfahren nach einem der Ansprüche 1 bis 4, wobei der Inhibitor des Endocannabinoid-abbauenden Enzyms ausgewählt ist aus der Gruppe bestehend aus URB597, PF-622, PF-750, PF-04457845, PF3845 und JZL184.

6. Verfahren nach Anspruch 5, wobei der Inhibitor des Endocannabinoid-abbauenden Enzyms ausgewählt ist aus der Gruppe bestehend aus JZL184 und URB597.

7. Verfahren nach einem der Ansprüche 1 bis 5, wobei der Sonic-Hedgehog-Inhibitor Cyclopamin ist und/oder wobei der Smad-Inhibitor ausgewählt ist aus der Gruppe bestehend aus Noggin, SB431542 und einer Kombination davon.

8. Zusammensetzung umfassend ein Agens, das in der Lage ist, das Signal, das von dem CB₁-Cannabinoidrezeptor vermittelt wird, in einer neuralen Stammzelle zu erhöhen, wobei die Erhöhung einen Anstieg der Anzahl von Ctip2(+)Satb2(-)-Zellen in Bezug auf die Basalspiegel von mindestens 5% zur Folge hat, wobei das Agens ausgewählt ist aus der Gruppe bestehend aus:
(i) einem CB₁-Cannabinoidrezeptor-Agonisten, der ausgewählt ist aus der Gruppe bestehend aus Anandamid, 2-Arachidonylglycerol (2-AG), N-Arachidonyldopamin, Arachidonyl-2'-chlorethylamid (ACEA), HU-210, JWH-073, WIN 55,212-2, Cannabinol, Tetrahydrocannabinol (THC), 11-Hydroxy-Δ⁹-tetrahydrocannabinol (11-OH-THC), Levonantradol, Dronabinol, AM-2201 (1-(5-Fluorpentyl)-3-(1-naphthoyl)indol), JWH-018 (1-Pentyl-3-(1-naphthoyl)indol), AM-678, 2-Arachidonylglycerolether (2-AGE, Noladin-Ether), Palmitoylethanolamid (PEA) und CP 55,940; und/oder
(ii) einem Inhibitor eines Endocannabinoid-abbauenden Enzyms
und einem Dorsalisierungsagens, das ausgewählt ist aus:
a) einem Sonic-Hedgehog-Inhibitor, der ausgewählt ist aus der Gruppe bestehend aus cyclischem Adenosin-3',5'-monophosphat, N6,O2'-Dibutyryl-Natriumsalz, AY 9944, KAAD-Cyclopamin, Cyclopamin, Forskolin, GANT58, GANT61, JK184, HPI-1, HPI-3, Jervin, SANT-1, GDC-0449, Robotnikinin und IPI-926; und
b) einem Smad-Inhibitor, der ausgewählt ist aus der Gruppe bestehend aus Noggin, Dorsomorphin, SB431542, LDN-193189, Lefty, Activin, TGF-beta und Kombinationen davon.

9. Zusammensetzung nach Anspruch 8, wobei der CB₁-Cannabinoidrezeptor-Agonist ausgewählt ist aus der Gruppe bestehend aus HU-210 und Arachidonyl-2'-chlorethylamid (ACEA).

10. Zusammensetzung nach einem der Ansprüche 8 und 9, wobei der Inhibitor eines Endocannabinoid-abbauenden Enzyms ausgewählt ist aus der Gruppe bestehend aus URB597, PF-622, PF-750, PF-04457845, PF3845 und JZL184.

11. Zusammensetzung nach Anspruch 10, wobei der Inhibitor eines Endocannabinoid-abbauenden Enzyms ausgewählt ist aus der Gruppe bestehend aus JZL184 und URB597.

12. Zusammensetzung nach einem der Ansprüche 8 bis 11, wobei der Sonic-Hedgehog-Inhibitor Cyclopamin ist und/oder wobei der Smad-Inhibitor ausgewählt ist aus der Gruppe bestehend aus Noggin, SB431542 und einer Kombination davon.

13. *In vitro*-Verwendung einer Zusammensetzung nach einem der Ansprüche 8 bis 12 zum Erhalten eines kortikospinalen oberen Motorneurons aus einer neuralen Stammzelle.

## Revendications

1. Un procédé *in vitro* pour l'obtention d'un neurone moteur supérieur corticospinal à partir d'une cellule souche nerveuse, dans lequel ledit procédé comprend la mise en contact d'une cellule souche nerveuse dans des conditions adéquates pour augmenter le signal médié par le récepteur cannabinoïde CB₁ dans ladite cellule et dans des conditions favorisant la dorsalisation de ladite cellule et dans lequel les conditions adéquates pour augmenter le signal médié par le récepteur cannabinoïde CB₁ impliquent une augmentation du nombre de cellules Ctip2+ Satb2- par rapport aux niveaux basaux d'au moins 5% et impliquent:
(i) la mise en contact de ladite cellule souche nerveuse avec un agoniste du récepteur cannabinoïde CB₁ choisi dans le groupe constitué par: anandamide, 2-arachidonoylglycérol (2-AG), N-arachidonoyl dopamine, arachidonyl-2'-chloroéthylamide (ACEA), HU-210, JWH-073, WIN 55,212-2, cannabinol, tétrahydrocannabinol (THC), 11-hydroxy-Δ⁹-tétrahydrocannabinol (11-OH-THC), lévonantradol, dronabinol, AM-2201 (1-(5-fluoropentyl)-3-(1-naphthoyl)indole), JWH-018 (1-pentyl-3-(1-naphthoyl)indole), AM-678, éther de 2-arachidonyle glycéryle (2-AGE, éther noladine), palmitoyléthanolamide (PEA) et CP 55,940; et / ou
(ii) l'introduction dans ladite cellule souche nerveuse d'un vecteur d'expression codant pour le récepteur cannabinoïde CB₁ pour augmenter l'expression du récepteur canabinoïde CB₁ au-dessus des niveaux de base et / ou
(iii) l'introduction dans ladite cellule souche nerveuse d'un vecteur d'expression codant pour une enzyme de synthèse de l'endocannabinoïde pour augmenter dans la cellule l'activité d'une enzyme de synthèse de l'endocannabinoïde au-dessus des niveaux de base et / ou
(iv) l'introduction dans ladite cellule souche nerveuse d'un agent qui inactive une enzyme de dégradation de l'endocannabinoïde choisi dans le groupe constitué d'un ARNsh, d'un ARNsi, d'un acide nucléique antisens et d'un ribozyme et / ou la mise en contact de ladite cellule souche nerveuse avec un inhibiteur de ladite enzyme de dégradation de l'endocannabinoïde,
et dans lequel les conditions favorisant la dorsalisation de ladite cellule impliquent la mise en contact de ladite cellule souche nerveuse avec un agent de dorsalisation choisi parmi :
a) un inhibiteur de sonic hedgehog choisi dans le groupe constitué de: adénosine monophosphate 3', 5'-cyclique, sel de sodium N6,02'-dibutyryl-, AY 9944, KAAD-cyclopamine, cyclopamine, forskoline, GANT58, GANT61, JK184, HPI-1 , HPI-3, jervine, SANT-1, GDC-0449, robotnikinine et IPI-926; et
b) un inhibiteur de Smad choisi dans le groupe constitué par: la noggine, la dorsomorphine, le SB431542, le LDN-193189, Lefty, Activine, TGF-bêta et leurs combinaisons.

2. Le procédé selon la revendication 1, dans lequel l'agoniste du récepteur cannabinoïde CB₁ est choisi dans le groupe constitué de: HU-210 et l'arachidonyl-2'-chloréthylamide (ACEA).

3. Le procédé selon l'une quelconque des revendications 1 ou 2, dans lequel l'enzyme de synthèse de l'endocannabinoïde est la diacylglycérol lipase.

4. Le procédé selon l'une quelconque des revendications 1 à 3, dans lequel l'enzyme de dégradation de l'endocannabinoïde est choisie dans le groupe constitué par l' hydrolase d'amide d'acide gras (FAAH) et la lipase de monoacylglycérol (MAGL).

5. Le procédé selon l'une quelconque des revendications 1 à 4, dans lequel l'inhibiteur de l'enzyme de dégradation de l'endocannabinoïde est choisi dans le groupe constitué de: URB597, PF-622, PF-750, PF-04457845, PF3845 et JZL184.

6. Le procédé selon la revendication 5, dans lequel l'inhibiteur de l'enzyme de dégradation de l'endocannabinoïde est choisi dans le groupe constitué de: JZL184 et URB597.

7. Le procédé selon l'une quelconque des revendications 1 à 5, dans lequel l'inhibiteur de sonic hedgehog est la cyclopamine et / ou l'inhibiteur de Smad est choisi dans le groupe constitué de: la noggine, le SB431542 et une combinaison de ceux-ci.

8. Une composition comprenant un agent capable d'augmenter le signal médié par le récepteur cannabinoïde CB₁ dans une cellule souche nerveuse, dans lequel ladite augmentation implique une augmentation du nombre de cellules Ctip2+ Satb2-par rapport aux niveaux de base d'au moins 5%, ledit agent étant choisi dans le groupe constitué de:
(i) un agoniste du récepteur cannabinoïde CB₁ choisi dans le groupe constitué par: anandamide, 2-arachidonoylglycérol (2-AG), N-arachidonoyl-dopamine, arachidonyl-2'-chloroéthylamide (ACEA), HU-210, JWH-073, WIN 55,212-2, cannabinol, tétrahydrocannabinol (THC), 11-hydroxy-Δ⁹-tétrahydrocannabinol (11-OH-THC), lévonantradol, dronabinol, AM-2201 (1-(5-fluoropentyl)-3-(1-naphthoyl)indole), JWH-018 (1-pentyl-3-(1-naphthoyl)indole), AM-678, éther de 2-arachidonyle glycéryle (2-AGE, éther noladine), palmitoyléthanolamide (PEA) et CP 55,940 ; et / ou
(ii) un inhibiteur d'une enzyme de dégradation de l'endocannabinoïde
et un agent de dorsalisation choisi parmi:
a) un inhibiteur de sonic hedgehog choisi dans le groupe constitué par: adénosine monophosphate 3',5'-cyclique, sel de sodium N6,02'-dibutyryl, AY 9944, KAAD-cyclopamine, cyclopamine, forskoline, GANT58, GANT61, JK184, HPI-1, HPI-3, jervine, SANT-1, GDC-0449, robotnikinine et IPI-926; et
b) un inhibiteur de Smad choisi dans le groupe constitué par: la noggine, la dorsomorphine, le SB431542, le LDN-193189, Lefty, Activine, TGF-bêta et leurs combinaisons.

9. La composition selon la revendication 8, dans laquelle l'agoniste du récepteur de cannabinoïde CB₁ est choisi dans le groupe constitué de: HU-210 et l'arachidonyl-2'-chloroéthylamide (ACEA).

10. La composition selon l'une quelconque des revendications 8 et 9, dans laquelle l'inhibiteur d'une enzyme de dégradation de l'endocannabinoïde est choisi dans le groupe constitué par: URB597, PF-622, PF-750, PF-04457845, PF3845 et JZL184.

11. La composition selon la revendication 10, dans laquelle l'inhibiteur d'une enzyme de dégradation de l'endocannabinoïde est choisi dans le groupe constitué par: JZL184 et URB597.

12. La composition selon l'une quelconque des revendications 8 à 11, dans laquelle l'inhibiteur de sonic hedgehog est la cyclopamine et / ou l'inhibiteur de Smad est choisi dans le groupe constitué par: la noggine, le SB431542 et une combinaison de ceux-ci.

13. Utilisation *in vitro* d'une composition selon l'une quelconque des revendications 8 à 12 pour l'obtention d'un neurone moteur supérieur corticospinal à partir d'une cellule souche nerveuse.


### SEQUENCE LISTING

<110> Centro de Investigación Biomedica en Red Enfermedades Neurodegenerativas (CIBERNED) Universidad Complutense de Madrid
<120> CORTICOSPINAL UPPER MOTOR NEURONS, METHODS AND COMPOSITIONS FOR DIFFERENTIATING NEURAL STEM CELLS BY MODULATING CB1 CANNABINOID RECEPTOR SIGNALING AND USES THEREOF
<130> P8325EP00
<160> 2
<170> PatentIn version 3.5
<210> 1
   <211> 19
   <212> DNA
   <213> Artificial Sequence
<220>
   <223> Clim1 forward primer
<400> 1
   accctcattc cccgttatt 19
<210> 2
   <211> 19
   <212> DNA
   <213> Artificial Sequence
<220>
   <223> Clim1 reverse primer
<400> 2
   tggctctcct accaccatc 19